# EUROPEAN PATENT SPECIFICATION

(11) **EP 1 371 732 B1**
(45) Date of publication and mention of the grant of the patent: **31.12.2008**
(21) Application number: 02700768.1
(22) Date of filing: 26.02.2002
(51) Int. Cl.: C12N 15/62, C12N 9/10, C07H 21/04, C08B 37/00, C12N 1/15, C12N 1/19, C12N 1/21, C12N 5/10, C12N 15/54, C12P 19/04, C12P 21/04, C12Q 1/68

(54) **FUSED PROTEIN HAVING BETA 1,2-N-ACETYLGLUCOSAMINYLTRANSFERASE II ACTIVITY AND PROCESS FOR PRODUCING THE SAME**
FUSIONSPROTEIN MIT BETA-1,2-N-ACETYLGLUCOSAMINYL-TRANSFERASE-II-AKTIVITÄT UND HERSTELLUNGSVERFAHREN DAFÜR
PROTEINE FUSIONNEE AYANT UNE ACTIVITE BETA 1,2-N-ACETYLGLUCOSAMINYLTRANSFERASE II ET PROCEDE DE PRODUCTION DE CETTE DERNIERE

(30) Priority: 26.02.2001 JP 2001049955; 21.08.2001 JP 2001250165
(43) Date of publication of application: 17.12.2003
(73) Proprietor: Toyo Boseki Kabushiki Kaisha, Osaka 530-0004 (JP); Fujiyama, Kazuhito, Suita-shi, Osaka 565-0824 (JP); Seki, Tatsuji, Toyonaka-shi, Osaka 560-0083 (JP)
(72) Inventor: FUJIYAMA, Kazuhito, Suita-shi, Osaka 565-0824 (JP); SEKI, Tatsuji, Toyonaka-shi, Osaka 560-0083 (JP); NISHIMURA, Shin-Ichiro, Sapporo-shi, Hokkaido 060-0009 (JP); NAKAGAWA, Hiroaki, Chuo-ku, Sapporo-shi, Hokkaido 060-0005 (JP); NISHIGUCHI, Susumu, Reseach Center of Toyo Boseki, Otsu-shi, Shiga 520-0243 (JP)
(74) Representative: Weber, Thomas
(86) International application number: PCT/JP2002/001695
(87) International publication number: WO 2002/068661

(56) References cited:
- EP-A- 0 286 239
- EP-A- 0 905 232
- WO-A-01/00848
- WO-A-96/21038
- DATABASE WPI Section Ch, Week 200168 Derwent Publications Ltd., London, GB; Class B04, AN 2001-599905 XP002286450 & JP 2001 178453 A (FUJIYAMA K), 3 July 2001 (2001-07-03)
- SASAKI K ET AL: "Expression cloning of cDNA encoding a human beta-1,3-N-acetylglucosam inyltransferase that is essential for poly-N-acetyllactosamine synthesis." PROCEEDINGS OF THE NATIONAL ACADEMY OF SCIENCES OF THE UNITED STATES OF AMERICA. UNITED STATES 23 DEC 1997, vol. 94, no. 26, 23 December 1997 (1997-12-23), pages 14294-14299, XP002930119 ISSN: 0027-8424
- LEROUGE P ET AL: "N-glycoprotein biosynthesis in plants: recent developments and future trends" PLANT MOLECULAR BIOLOGY, NIJHOFF PUBLISHERS, DORDRECHT, NL, vol. 38, 1998, pages 31-48, XP002140796 ISSN: 0167-4412
- TAKAHASHI S ET AL: "A NEW BETA-1,2-N-ACETYLGLUCOSAMINYLTRANSFERASE THAT MAY PLAY A ROLE IN THE BIOSYNTHESIS OF MAMMALIAN O-MANNOSYL GLYCANS" GLYCOBIOLOGY, IRL PRESS,, GB, vol. 11, no. 1, 2001, pages 37-45, XP002907899 ISSN: 0959-6658
- BENDIAK B ET AL: "CONTROL OF GLYCOPROTEIN SYNTHESIS PURIFICATION OF UDP-N ACETYLGLUCOSAMINE ALPHA-D MANNOSIDE BETA-1-2 N ACETYLGLUCOSAMINYLTRANSFERASE II FROM RAT LIVER" JOURNAL OF BIOLOGICAL CHEMISTRY, vol. 262, no. 12, 1987, pages 5775-5783, XP002286449 ISSN: 0021-9258
- TAN J. ET AL.: 'The human UDP-N-acetylglucosamine: alpba-6-D-mannoside-beta-1,3-N-acetylglucos aminyltransferase II gene (MGAT2). Cloning of genomic DNA, localization to chromosome 14q21, expression in insect cells and purification of the recombinant protein' EUR. J. BIOCHEM. vol. 231, 1995, pages 317 - 328, XP002953573
- WELLER U. ET AL.: 'Expression of active streptolysin O in escherichia coli as a maltose- binding-protein-streptolysin-O fusion protein. The N- terminal 70 amino acids are not required for hemolytic activity' EUR. J. BIOCHEM. vol. 236, 1996, pages 34 - 39, XP002953574
- RECK F. ET AL.: 'Synthesis of pentasaccharide analogues of the N-glycan substrates of N-acetylglucosaminyltransferase III, IV and V using tetrasaccharide precursors and recombinant beta-(1->2)-N-acetylglucosaminyltransferase II.' CARBOHYDR. RES. vol. 275, 1995, pages 221 - 229, XP004021876
- RECK F. ET AL.: 'Synthesis of tetrasaccharide analogues of the N-glycan substrate of beta-(1->2)-N-acetylglucosaminyltransferase II using trisaccharide precursors and recombinant beta-(1->2)-N-acetylglucosaminyltransferase I.' CARBOHYDR. RES. vol. 259, 1994, pages 93 - 101, XP002953575
- SCHACHTER H.: 'Mini review: The yellow brick road to branched complex N-glycans' GLYCOBIOLOGY vol. 1, no. 5, 1991, pages 453 - 461, XP002953576
- MORAN D.G. ET AL: 'Expression of glycoprotein-processing enzymes in Escherichia coli as fusion proteins' WORLD JOURNAL OF MICROBIOLOGY & BIOTECHNOLOGY vol. 13, 1997, pages 265 - 267
- SHANG J. ET AL: 'Molecular cloning and expression of Galbeta1,3GalNAc alpha2,3-sialyltransferase from human liver' EUR. J. BIOCHEM vol. 265, 1999, pages 580 - 588
- MORAN D.G. ET AL: 'Characterization of Recombinant Human Man9-Mannosidase expressed in Escherichia coli' JOURNAL OF FERMANTATION AND BIOENGINEERING vol. 86, no. 3, 1998, pages 277 - 283
- FUKUOKA Y. ET AL: 'Expression of biologically active C3a as fusion proteins' IMMUNOLOGY LETTERS vol. 38, 1993, pages 153 - 158

## Description

The present invention relates to a method for the inexpensive and efficient production of β1,2-N-acetyl glucosaminyltransferase II, which is a glycoprotein processing enzyme, and in particular to a fusion protein of a maltose-binding protein and β1,2-N-acetylglucosaminyltransferase II, as well as a method for producing this protein in *E. coli* cells. The present invention furthermore relates to modification of sugar chain structures attached to glycoprotein.

### BACKGROUND ART

Sugar chains comprising various monosaccharides linked by glycoside bonds exist in the form of intracellular organelle constituents, cell surface constituents, secretory proteins, and the like in cells. These sugar chain structures not only differ in ways specific to species and tissue, but also differ depending on development stage, disease, and the like even within the same species and tissue. It has thus become apparent that sugar chains function to provide proteins with physical properties conventionally attributed to proteins, such as thermal stability, hydrophilicity, electrical charge, and protease resistance, and that they also play a role in inter-cellular recognition such as that involved in development/differentiation, the nervous system, the immune system, and cancer metastasis, making them the subject of considerable recent scrutiny for potential application to various fields such as medicinal products.

These sugar chains are synthesized by glycosyltransferases within organisms. Glycosyltransferases are enzymes which transfer sugar from sugar nucleotides as sugar donors to acceptor sugar chains and extend the sugar chain. The specificity of sugar acceptor is highly stringent, it being commonly said that one glycoside bond is formed by one corresponding glycosyltransferase. These glycosyltransferases are important enzymes in terms of their use in glycobiological research, particularly the simple synthesis of useful sugar chains and the repair of natural sugar chains. However, the amounts of naturally occurring glycosyltransferase are limited, making it difficult to consistently provide large amounts for practical purposes.

β1,2-N-acetylglucosaminyltransferase II (GnTII) is an important enzyme involved in the formation of complex type sugar chains in glycoproteins, and has the action of transferring GlcNAc by means of β1-2 linkages to the acceptor Manα1-6(GlcNAcβ1-2Manα1-3)Manβ1-4GlcNAcβ1-4GlcNAc-R using at least UDP-GlcNAc as the sugar donor, so as to produce GlcNAcβ1-2Manα1-6(GlcNAcβ1-2Manα1-3)Manβ1-4GlcNAcβ1-4GlcNAc-R (where R is asparagine residue, peptide, protein, or a low- or high-molecular weight compound which does not inhibit the activity of other glycosyltransferases). The consistent supply of such enzymes would be desirable because they are key enzymes in the formation of complex type sugar chains.

β1,2-N-acetylglucosaminyltransferase II has been purified from the organs of various species of animals. However, because organs are not readily available in large quantities, and because attempts at purification resulting in a single protein have proven to be extremely laborious, recombinant production is a promising alternative.

The cDNA coding for such enzymes has been isolated from various biological materials. For example, human β1,2-N-acetylglucosaminyltransferase II has been isolated by Tan et al. from leukocytes (Eur. J. Biochem., 231: 317-328 (1995)). It has also been isolated by R. Strasser et al. from Arabidopsis (Glycoconj. J., 16:787-791 (2000)).

The expression of proteins using microbes such as bacteria or yeast is beneficial in terms of cost for the mass production of recombinant proteins. However, as most proteins expressed in microbial expression systems occur in the form of insoluble inclusion bodies, and must be solubilized and renatured before purification, this cannot be considered an efficient option. In addition, even enzymes expressed in soluble form must undergo several purification processes, including various types of chromatographic treatment, in order to achieve a high degree of purification. Because this is considerably time-consuming and costly, it is not a satisfactory expression system for commercial industrial purposes.

An example of a method for expressing large amounts of the target protein and easily purifying the expression product is to express the target protein in the form of a fusion protein with glutathione-S-transferase (GST), protein A, or the like. In this method, fusion proteins containing GST can be readily purified by affinity column chromatography using glutathione as the ligand, and fusion proteins containing protein A can be readily purified by affinity column chromatography using IgG as the ligand. However, even in the case of above GST method the high possibility of producing inclusion body still remains. For these reasons, it is difficult to achieve a consistent supply of large quantities of β1,2-N-acetylglucosaminyltransferase II.

As noted above, most proteins in higher life forms occur in the form of glycoproteins, and their sugar chain structure is deeply involved in the recognition between cells and metabolic rates such as the absorption and degradation of substances in organisms. For that reason, when genes coding for physiologically active proteins derived from animals are expressed using yeast or plant hosts, for example, the sugar chain structure of the resulting glycoproteins differs from that of the original from the animal, often resulting in lower physiological activity (and sometimes no activity) or a higher degradation rate. It would be extremely useful if there was a method allowing such altered sugar chains to be modified back into the original sugar chain from the animal. Also, modifying sugar chains into ones that are different from the original bound sugar chains is expected to be of use in strengthening physiological functions or modifying physiological activity. Altering the expressing host or modifying the host through the introduction of a glycosyltransferase gene will alter the sugar chains binding to the protein targeted for expression but will not necessarily result in only the desired changes. A better method would be to modify the sugar chains of obtained glycoproteins in vitro. Several methods making use of transglycosylation by endoglycosidase have been proposed as such methods (such as Japanese Unexamined Patent Application (Kokai) 5-64594). However, endoglycosidase transglycosylation generally results in poor yields on sugar acceptors, and does not permit the efficient modification of sugar chains. Another method is to use exoglycosidase and a glycosyltransferase (Eur. J. Biochem., 191:75-73 (1990)), but the most that can be done is to modify the nonreducing terminal sugar residues, and this method thus cannot be considered to modify the entire sugar chain. There is also a method for using endoglycosidase and a glycosyltransferase (J. Am. Chem., Soc., 119:2114-2118 (1997)). In this method, hydrolysis is carried out with the endoglycosidase, and the sugar chains are then extended with the glycosyltransferase at the nonreducing terminal of the N-acetylglucosamine residues left on the protein, resulting in conversion to glycoproteins bound Sialyl-Lewis x tetrasaccharides thereto, but the bound sugar chains are the nonreducing terminal part of the glycoprotein sugar chains, making this method inadequate for modifying the entire sugar chain. One reason that current methods for modifying sugar chains with the use of exo- or endo- glycosidases and giycosyitransferases have not been adequate is that it has been difficult to ensure a stable supply of large amounts of β1,2-N-acetylglucosaminyltransferase II, the key enzyme for conversion.

An object of the invention is therefore to ensure the inexpensive and efficient supply of protein having β1,2-N-acetylglucosaminyltransferase II activity.

Another object of the invention is to modify sugar chain structures attached to glycoprotein.

### DISCLOSURE OF THE INVENTION

As a result of extensive research to overcome the drawbacks described above, the inventors discovered that when genetically engineered β1,2-N-acetylglucosaminyl transferase II is expressed in the form of a fusion protein with a sugar-binding protein such as a maltose-binding protein (MBP) in *E. coli,* β1,2-N-acetylglucosaminyltransferase II can be obtained in the form of a soluble protein, the protein can be readily purified by affinity chromatography using the specific affinity of sugar-binding proteins, and the resulting fusion protein has β1,2-N-acetylglucosaminyltransferase II activity (GnTII activity).

The inventors also discovered that GnTII can be obtained from the fusion protein using a protease that specifically cleaves the sequence at the site where the sugar-binding protein and GnTII are fused.

The inventors furthermore discovered that sugar chains on glycoprotein can be converted in vitro using GnTII capable of being consistently supplied in large amounts.
It was also discovered that such sugar chains on glycoprotein can be converted using immobilized enzymes. That is, the invention encompasses the following inventions:
(1) A recombinant fusion protein of a maltose-binding protein and β1,2-N-acetylglucosaminyltransferase II.
(2) The fusion protein of item (1), wherein the β1,2-N-acetylglucosaminyltransferase II is derived from humans.
(3) The fusion protein of item (1), wherein the β1,2-N-acetylglucosaminyltransferase II is (a) a protein comprising the amino acid sequence in SEQ ID NO. 2, or (b) a protein with β1,2-N-acetylglucosaminyl transferase II activity, comprising the amino acid sequence in (a) above with one or more amino acids deleted, substituted, or added.
(4) The fusion protein of item (3), wherein the β1,2-N-acetylglucosaminyltransferase II comprises at least the amino acid sequence 20-447 in the amino acid sequence in SEQ ID NO:2.
(5) The fusion protein of item (1), wherein the β1,2-N-acetylglucosaminyltransferase II comprises an amino acid sequence in which amino acids corresponding to part or all of the protein transmembrane domain have been deleted.
(6) The fusion protein of item (1), comprising a protease recognition site between the sugar-binding protein and the β1,2-N-acetylglucosaminyl transferase II.
(7) DNA coding for a fusion protein according to any one of items (1) through (6).
(8) An expression vector comprising DNA according to item (7).
(9) A transformant resulting from transformation with an expression vector according to item (8).
(10) A method for producing a maltose-binding protein/β1,2-N-acetylglucosaminyltransferase II fusion protein, comprising the following steps of:
   (1) transforming *E. coli* using an expression vector to which DNA coding for a maltose-binding protein and DNA coding for β1,2-N-acetylglucosaminyltransferase II have been ligated in such a way that the two proteins are expressed in the form of a fusion protein under the control of a promoter capable of functioning in *E*. *coli;*
   (2) cultivating the resulting transformants to produce a fusion protein of the maltose-binding protein and the β1,2-N-acetylglucosaminyltransferase II; and
   (3) isolating the fusion protein from the resulting culture.
(11) The method of item (10), wherein the β1,2-N-acetylglucosaminyltransferase II is derived from humans.
(12) The method of item (11), wherein the DNA coding for the β1, 2-N-acetylglucosaminyltransferase II comprises a nucleotide sequence coding for at least the amino acid sequence 29-447 in the amino acid sequence in SEQ ID NO:2.
(13) The method of item (11), wherein the DNA coding for the β1,2-N-acetylglucosaminyltransferase II comprises at least the nucleotide sequence 85-1341 in the nucleotide sequence in SEQ ID NO. 1.
(14) The method of item (10), wherein the DNA coding for the β1,2-N-acetylglucosaminyltransferase II comprises a nucleotide sequence coding for an amino acid sequence in which amino acids corresponding to part or all of the protein transmembrane domain have been deleted.
(15) The method of item (10), wherein the DNA coding for the β1,2-N-acetylglucosaminyltransferase II comprises the nucleotide sequence in SEQ ID NO. 1 from which amino acids corresponding to part or all of the protein transmembrane domain have been deleted.
(16) The method of item (10), wherein the DNA coding for the maltose-binding protein is derived from pMAL-p2 or pMAL-c2.
(17) The method of item (10), wherein the fusion protein is isolated in the presence of divalent metal ions from the culture obtained in step (3).
(18) The method of item (17), wherein the divalent metal is manganese.
(19) A method for producing β1,2-N-acetyl glucosaminyltransferase II, comprising the step of isolating the β1,2-N-acetylglucosaminyltransferase II after eliminating the maltose-binding protein portion from the fusion protein obtained by a method according to any of items (10) through (18).
(20) The method of item (19), characterized in that the DNA coding for the sugar-binding protein comprises a nucleotide sequence coding for a protease recognition site on the C terminal end of the protein, and the sugar-binding protein portion is eliminated .from the fusion protein through the action of a protease.
(21) The method of item (20), wherein the protease is blood coagulation factor Xa.
(22) A method for converting sugar chains on glycoproteins to complex type sugar chains, comprising steps (1) through (3) below:
   (1) allowing a glycosidase to act on glycoprotein sugar chains;
   (2) allowing β1,2-N-acetylglucosaminyltransferase I to act, in the presence of UDP-GlcNAc, on the glycoproteins obtained in step (1); and
   (3) allowing β1,2-N-acetylglucosaminyltransferase II, being a recombinant fusion protein according to any one of items 1 through 6, to act, in the presence of UDP-GlcNAc, on the glycoproteins obtained in step (2).
(23) The method of item (22), characterized in that at least one kind of glycosyltransferase is furthermore allowed to act after step (3).
(24) The method of item (23), wherein the glycosyltransferase is at least one selected from sialyltransferase, fucosyltransferase, galactosyltransferase, and N-acetylglucosaminyltransferase.
(25) The method of item (23), wherein at least one glycosyltransferase is an immobilized enzyme.
(26) The method of item (22), wherein the glycosidase is at least one selected from galactosidase, N-acetylglucosaminidase, fucosidase, sialidase, xylosidase, and mannosidase.
(27) The method of item (22), wherein at least one of the glycosidase, β1,2-N-acetylglucosaminyltransferase I, or β1,2-N-acetylglucosaminyltransferase II is an immobilized enzyme.
(28) The method of item (22), wherein the glycoprotein is naturally derived.
(29) The method of item (22), wherein the glycoprotein is recombinant.
(30) The method of item (22) comprising steps (1) through (4) below:
   (1) allowing a glycosidase to act on glycoprotein sugar chains;
   (2) allowing β1,2-N-acetylglucosaminyltransferase I to act, in the presence of UDP-GlcNAc, on the glycoproteins obtained in step (1);
   (3) allowing α-mannosidase to act on the glycoproteins obtained in step (2); and
   (4) allowing β1,2-N-acetylglucosaminyltransferase II, being a recombinant fusion protein according to any one of items 1 through 6, to act, in the presence of UDP-GlcNAc, on the glycoproteins obtained in step (3).
(31) The method of item (30), characterized in that at least one kind of glycosyltransferase is furthermore allowed to act after step (4).
(32) The method of item (31), wherein the glycosyltransferase is at least one selected from sialyltransferase, fucosyltransferase, galactosyltransferase, and N-acetylglucosaminyltransferase.
(33) The method of item (31), wherein at least one glycosyltransferase is an immobilized enzyme.
(34) The method of item (30), wherein the glycosidase is at least one selected from galactosidase, N-acetylglucosaminidase, fucosidase, sialidase, xylosidase, and mannosidase.
(35) The method of item (30), wherein the glycosidase is α-mannosidase.
(36) The method of item (30), wherein the glycosidase is α1,2-mannosidase.
(37) The method of item (30), wherein the α-mannosidase is α-mannosidase II.
(38) The method of item (30), wherein at least one of the glycosidase, β1,2-N-acetyl glucosaminyltransferase I, α-mannosidase, or β1,2-N-acetylglucosaminyltransferase II is an immobilized enzyme.
(39) The method of item (30), wherein the glycoprotein is naturally derived.
(40) The method of item (30), wherein the glycoprotein is recombinant.
(41) A method for converting sugar chains on glycoproteins to complex type sugar chains, comprising the following steps (1) through (3):
   (1) allowing β1,2-N-acetylglucosaminyl transferase I to act, in the presence of UDP-GIcNAc, on glycoproteins having a structure wherein part or all of the sugar chain structures on the glycoproteins serve as the substrate for the β1,2-N-acetylglucosaminyltransferase I;
   (2) allowing α-mannosidase to act on the glycoproteins obtained in step (1); and
   (3) allowing β1,2-N-acetylglucosaminyltransferase II being a recombinant fusion protein according to any one of items 1 through 6 to act, in the presence of UDP-GIcNAc, on the glycoproteins obtained in step (2).
(42) The method of item (41) for converting sugar chains on glycoproteins to complex type sugar chains, characterized in that at least one kind of glycosyltransferase is furthermore allowed to act after step (3).
(43) The method of item (42), wherein the glycosyltransferase is at least one selected from sialyltransferase, fucosyltransferase, galactosyltransferase, and N-acetylglucosaminyltransferase.
(44) The method of item (42), wherein at least one glycosyltransferase is an immobilized enzyme.
(45) The method of item (41), wherein the α-mannosidase is α-mannosidase II.
(46) The method of item (41), wherein at least one of the β1,2-N-acetylglucosaminyltransferase I, α-mannosidase, or β1,2-N-acetylglucosaminyltransferase II is an immobilized enzyme.
(47) The method of item (41), wherein the glycoprotein is naturally derived.
(48) The method of item (41), wherein the glycoprotein is recombinant.
(49) A method for converting hybrid-type sugar chains on glycoproteins to complex-type sugar chains, comprising steps (1) and (2) below:
   (1) allowing a glycosidase to act on hybrid-type sugar chains of glycoproteins; and
   (2) allowing β1,2-N-acetylglucosaminyltransferase II, being a recombinant fusion protein according to any one of items 1 through 6, to act, in the presence of UDP-GlcNAc, on the glycoproteins obtained in step (1).
(50) The method of item (49), characterized in that at least one kind of glycosyltransferase is furthermore allowed to act after step (2).
(51) The method according to item (50), wherein the glycosyltransferase is at least one selected from sialyltransferase, fucosyltransferase, galactosyltransferase, and N-acetylglucosaminyltransferase.
(52) The method of item (50), wherein at least one glycosyltransferase is an immobilized enzyme.
(53) The method of item (49), wherein the glycosidase is at least one selected from mannosidase, xylosidase, fucosidase, and β1,4-N-acetylglucosaminidase.
(54) The method of item (49), wherein at least one of the glycosidase or β1,2-N-acetyl glucosaminyltransferase II is an immobilized enzyme.
(55) The method of item (49), wherein the glycoprotein is naturally derived.
(56) The method of item (49), wherein the glycoprotein is recombinant.

### BRIEF DESCRIPTION OF THE DRAWINGS

Figure 1 is an outline of the preparation of a vector in the invention (Example 2);
Figure 2 shows the results of HPLC analysis indicating the reactivity of the fusion protein solution (MBP-hGnTII) obtained in Example 3. Figure 2A gives the results of analysis for the standard pyridylaminated oligosaccharides (Takara Shuzo) represented by Formulas 2 and 3, and Figure 2B gives the results from 3 hours of reaction using the pyridylaminated oligosaccharide represented by Formula 2 as the substrate (Example 4);
Figure 3 shows the optimal reaction temperature for the MBP-hGnTII obtained in Example 3 (Example 6). The activity at 40°C was considered 100%;
Figure 4 shows the thermal stability of the MBP-hGnTII obtained in Example 3 (Example 7). The activity at 10°C was considered 100%;
Figure 5 shows the optimal pH for the MBP-hGnTII obtained in Example 3 (Example 8). The activity in cacodylate buffer (pH 7.0) was considered 100%;
Figure 6 shows the pH stability of the MBP-hGnTII obtained in Example 3 (Example 9). The activity in glycine buffer (pH 8.8) was considered 100%;
Figure 7 shows the sugar chain analysis of samples obtained in the steps in Example 10, as determined by normal phase HPLC;
Figure 8 shows the results of mass spectrum analysis of the main peak in step 4 in Example 10;
Figure 9 illustrates an embodiment of the invention as described on items (30) through (40);
Figure 10 illustrates an embodiment of the invention as described on items (41) through (48);
Figure 11 illustrates an embodiment of the invention as described on items (22) through (29);
Figure 12 illustrates an embodiment of the invention as described on items (49) through (56); and

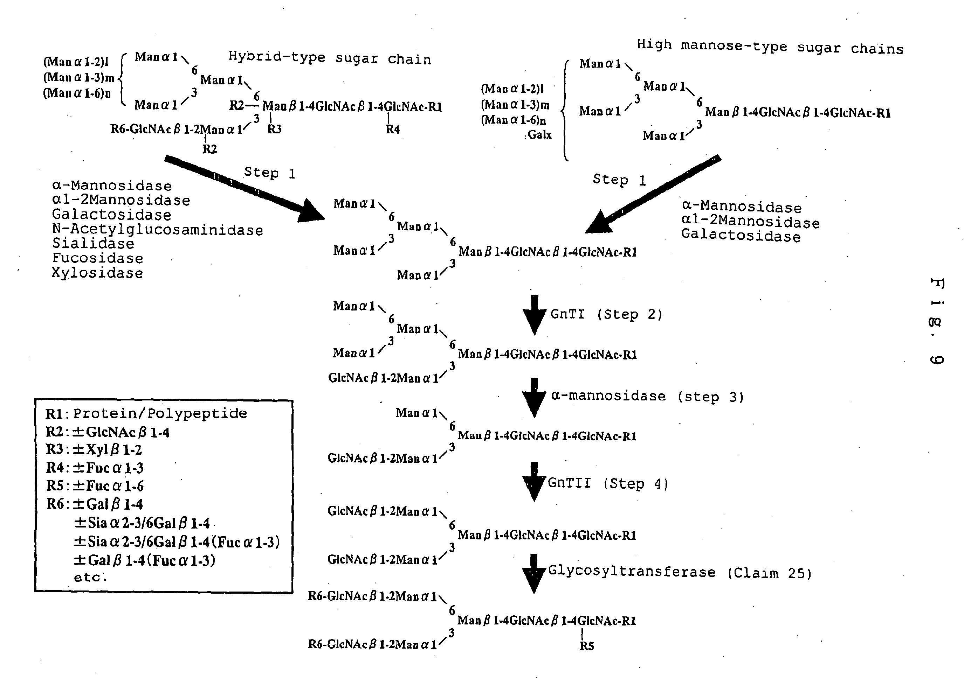

In Figures 9 through 12, the symbols l, m, n, and x represent integers from 0 to 20.

### DETAILED DESCRIPTION OF THE INVENTION

The present invention is characterized in that proteins with GnTII activity are reconstructed in the form of fusion proteins of GnTII and a sugar-binding protein, the fusion protein being a soluble protein.

The present invention is also characterized by the fact that GnTII can be expressed as a fusion protein with a sugar-binding protein in *E. coli* cells to produce GnTII in the form of a soluble protein, and the fact that the specific affinity of the sugar-binding protein can be exploited to allow the fusion protein and GnTII to be readily purified in large amounts.

The present invention is furthermore characterized in that sugar chains on glycoprotein can be converted using a glycosidase and glycosyltransferase.

### Fusion Protein of the Invention

In the present invention, the fusion protein of a sugar-binding protein such as MBP and GnTII (sometimes abbreviated as MBP-GnTII below for fusion proteins containing maltose-binding protein) may be in any form, as long as it retains the specific affinity of the sugar-binding protein, that is, the high affinity for maltose or oligosaccharides and polysaccharides containing maltose segment, as well as the enzyme activity of GnTII, that is, the activity in transferring GlcNAc from UDP-GlcNAc as the sugar donor via β1-2 linkages to the acceptor Manα1-6(GlcNAcβ1-2Manα1-3)Manβ1-4GlcNAcβ1-4GlcNAc-R (where R is asparagine residue, peptide, protein, or low- or high-molecular weight compound which does not inhibit the activity of other glycosyltransferases), so as to produce GlcNAcβ1-2Manα1-6(GlcNAcβ1-2Manα1-3)Manβ1-4GlcNAcβ1-4GlcNAc-R. Furthermore, as indicated in Formula 1, GnTII obtained after the elimination of the sugar-binding protein portion has at least inherent enzyme activity, that is, activity in transferring GlcNAc from UDP-GlcNAc as the sugar donor via β1-2 linkages to the acceptor Manα1-6 (GlcNAcβ1-2Manα1-3) Manβ1-4GlcNAcβ1-4GlcNAc-R, so as to produce GlcNAcβ1-2Manα1-6(GlcNAcβ1-2Manα1-3)Manβ1-4GlcNAcβ1-4GlcNAc-R.

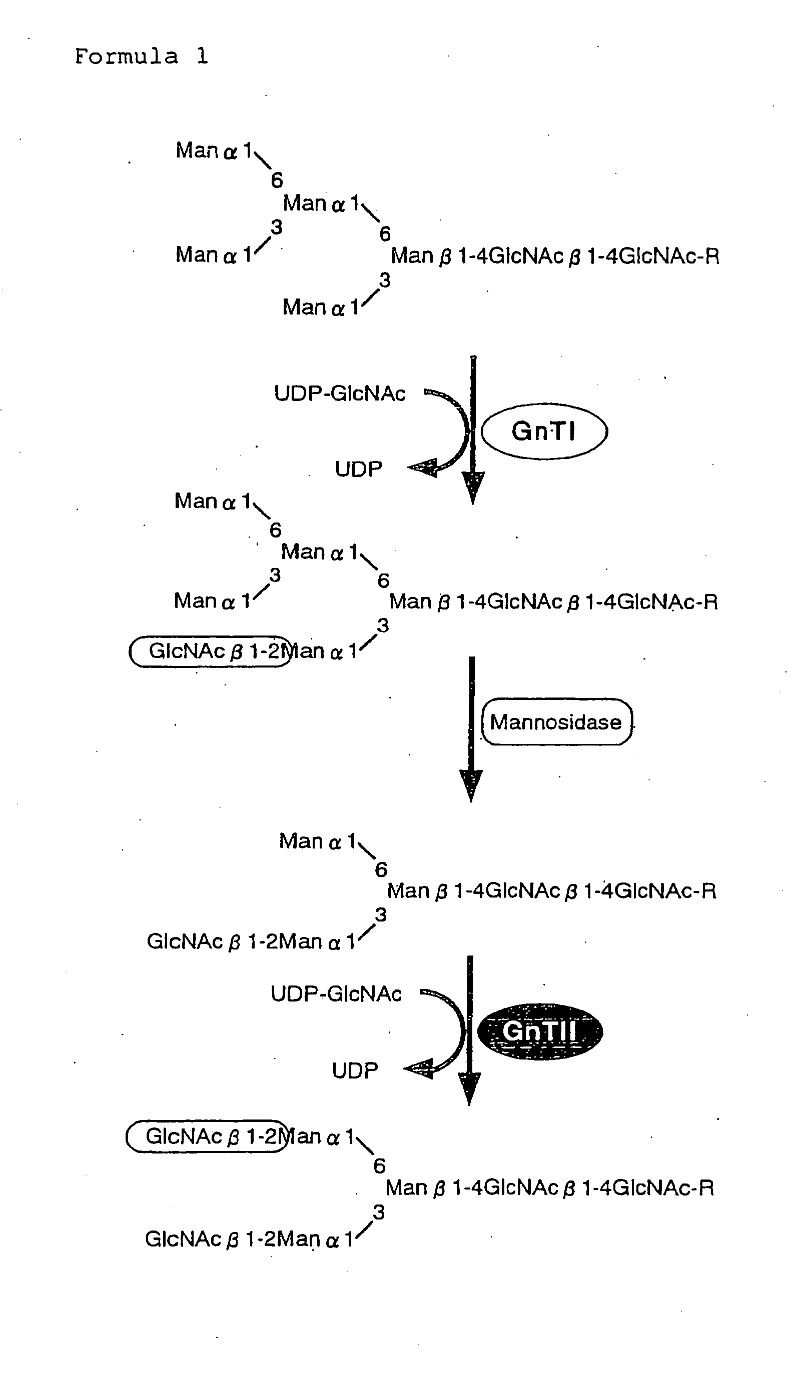

The GnTII will preferably be linked to the C terminal of the sugar-binding protein in the fusion protein. Even more preferably, the fusion protein will have a structure allowing the sugar-binding protein and GnTII to be readily cleaved by enzymatic or chemical means at the fusion site.

Sugar-binding proteins are proteins having high affinity for monosaccharides as well as oligosaccharides and polysaccharides, examples of which include various lectins, maltose-binding protein, cellulose-binding protein, and chitin-binding protein. Maltose-binding protein, cellulose-binding protein, chitin-binding protein, and the like are preferred, and maltose-binding protein (MBP) is especially desirable.

The sugar-binding proteins can be derived from any species, but are preferably from prokaryotes such as bacterial cells, and even more preferably from *E. coli* cells.

MBP need not necessarily have the complete sequence, and may include part of the sequence, provided that they have sites with affinity specific to maltose or oligosaccharides with maltose segment (such as maltotriose) or polysaccharides with maltose segment (such as amylose). Examples include proteins which have the known amino acid sequence with one or more amino acids deleted, substituted, or added, and has a domain with affinity to maltose or oligosaccharides with maltose segment (such as maltotriose) or polysaccharides with maltose segment (such as amylose).

DNA derived from pMAL-p2 or pMAL-c2 (both from New England Biolabs) were used as the genes DNA coding for MBP in the present invention.

The sugar-binding proteins of the present invention should especially include an amino acid sequence which is recognized by a protease with high substrate specificity at the C terminal of the protein. Examples of such proteases include factor Xa, thrombin, and renin. The protease recognition sequence is preferably the factor Xa recognition sequence, that is, Ile-Glu-gly-Arg (SEQ ID NO. 3). Such a protease recognition sequence can be introduced to the C terminal of the sugar-binding protein so as to allow the action of the protease after purification of the MBP-GnTII to readily eliminate the MBP.

In a preferred embodiment, the fusion protein will include a spacer sequence consisting of about 5 to 15 amino acid residues between the sugar-binding protein moiety and the protease recognition site. The spacer is intended to put some distance between the sugar-binding protein and the GnTII so as to deduce the possibility of intramoiecular interaction in the fusion protein, and to enhance the affinity for maltose or amylose in the MBP portion of MBP-GnTII fusion proteins. pMAL-p2 or pMAL-c2 (by New England Biolabs) including the spacer sequence in SEQ ID NO:9 can be used in the present invention.

GnTII which is derived from any species is applicable to the present invention but is preferably from mammals and more preferably from humans. Furthermore, in the present invention, the GnTII need not contain the full length GnTII sequence, and may include part of the sequence, provided that the GnTII activity is preserved. For example, it may be (a) a protein comprising the amino acid sequence in SEQ ID NO. 2 or (b) a protein that comprises the amino acid sequence in (a) with one or more amino acids deleted, substituted, or added, and that has β1,2-N-acetylglucosaminyltransferase II activity.

Because GnTII is a membrane protein in the Golgi apparatus, a particularly desirable embodiment of the invention features the use of GnTII lacking part or all of its highly hydrophobic transmembrane domain, in the interests of allowing the MBP-GnTII to be produced in the form of a soluble protein. Specifically, the invention should include the amino acid sequence from at least 29-447 in the amino acid sequence in SEQ ID NO. 2.

It may also be a protein that comprises the amino acid sequence from at least 29-447 in the amino acid sequence of SEQ ID NO. 2, with one or more amino acids deleted, substituted, or added, and that has β1,2-N-acetylglucosaminyltransferase II activity.

### Preparation of the Fusion Protein of the Invention

In the present invention, the sugar-binding protein-GnTII fusion protein can be obtained by cultivating, in a suitable medium, the transformant, which has been obtained upon the transformation of *E. coli* with an expression vector comprising chimera DNA ligated in such a way - namely, in-frame - that the DNA coding for the sugar-binding protein and the DNA coding for the GnTII are transcribed and translated in the form of a fusion protein having the properties described above.

In the present invention, the DNA coding for GnTII may be derived from any species, but is preferably from mammals, and even more preferably from humans. The DNA coding for the GnTII in the present invention need not include the entire coding region of the GnTII, and may include a part of the coding region, provided that the transcription/translation product retains GnTII activity. Because GnTII is a membrane protein in the Golgi apparatus, a particularly desirable embodiment of the invention features the use of GnTII lacking the region coding for the highly hydrophobic transmembrane domain, in the interests of allowing the sugar-binding protein-GnTII to be produced in the form of a soluble protein.

Based on the known nucleotide sequence coding for GnTII (such as Eur. J. Biochem., 231:317-328 (1995)) or the like, the DNA coding for the GnTII can be prepared by any known method. For example, a pair of suitable oligonucleotide primers covering part or all of the GnTII coding region can be synthesized based on the known GnTII nucleotide sequence, and the total RNA or poly A⁽⁺⁾RNA or chromosomal DNA extracted from cells or tissue expressing GnTII can be used as template in RT-PCR or PCR to clone the DNA. A suitable restriction enzyme recognition sequence can be added to the terminal of the oligonucleotide primers that are used, in order to facilitate subsequent cloning to the vector.

Alternatively, a suitable oligonucleotide probe can be synthesized based on the known GnTII nucleotide sequence, a cDNA library can be prepared in the usual manner from cells or tissue expressing the GnTII, and the DNA coding for GnTII can be obtained from the library by plaque (or colony) hybridization.

Furthermore, antibody is prepared in the usual manner by using partially or completely purified GnTII as antigen, the DNA coding for the GnTII can be cloned from cDNA library prepared in the usual manner from cells or tissue expressing the GnTII by using the antibody.

Alternatively, DNA can be synthesized based on the known GnTII nucleotide sequence using a DNA/RNA automatic synthesizer in such a way that part of the sense strand partial sequence overlaps part of the antisense strand partial sequence, and the longer partial sequences are repeatedly obtained in the form of double-stranded DNA by PCR, giving the desired DNA sequence.

In a preferred embodiment of the invention, the DNA is comprised of part or all of the DNA coding for human β1,2-N-acetylglucosaminyltransferase II (hGnTII). Examples include DNA comprising a nucleotide sequence coding for the amino acid sequence at least 29-447 in the amino acid sequence in SEQ ID NO. 2, and preferably the nucleotide sequence at least 85-1341 in the nucleotide sequence in SEQ ID NO. 1. Alternatively, the DNA coding for hGnTII may be DNA comprising a nucleotide sequence coding for an amino acid sequence in which an amino acid sequence corresponding to part or all of the transmembrane domain of the protein has been deleted, and preferably comprises a nucleotide sequence in which a nucleotide sequence coding for an amino acid sequence corresponding to part or all of the transmembrane domain of the protein has been deleted. Based on hydrophobic plot results, the transmembrane domain of hGnTII is assumed to be the portion represented by amino acid sequence 10-28 in the amino acid sequence of SEQ ID NO. 2. The DNA coding for the domain is therefore preferably the nucleotide sequence 28-84 in the nucleotide sequence in SEQ ID NO:1.

Of course, the DNA coding for the hGnTII may be DNA comprising a nucleotide sequence coding for all of the amino acid sequence in SEQ ID NO. 2, and is preferably DNA comprising all of the nucleotide sequence in SEQ ID NO. 1.

Examples of DNA coding for the sugar-binding protein in the present invention include DNA coding for the various proteins described above, and preferably DNA coding for maltose-binding protein, cellulose-binding protein, or chitin-binding protein, especially DNA coding for maltose-binding protein.

The DNA may be from any species, but is preferably from prokaryotes such as bacterial cells, especially *E. coli* cells.

The DNA coding for the MBP need not necessarily include the entire coding region, and may include part of the coding region, provided that it codes for a translation product having affinity for maltose, oligosaccharides with maltose segment (such as maltotriose), or polysaccharides with maltose segment (such as amylose). Similarly, the sugar-binding protein need not necessarily include the entire coding region, and may include part of the coding region, provided that it codes for a translation product with affinity for the binding sugar.

MBP derived from *E. coli* is secretory protein localized in periplasm, and the initial translation product includes a signal peptide at the N terminal. DNA coding for MBP in the present invention may or may not include a nucleotide sequence coding for a signal peptide (signal codon) capable of functioning in *E. coli* hosts. Because *E*. *coli* is a gram negative bacterium with an outer membrane outside of the cell wall, even when the DNA coding for MBP includes a signal codon, there is little possibility of the expressed MBP-GnTII being secreted in media. However, if the MBP-GnTII accumulates in the periplasmic space, spheroplasts can be formed without completely rupturing the cells, thereby allowing the fusion protein to be recovered, and thus making subsequent purification easier to manage. In such cases, however, the transmembrane domain of GnTII should be eliminated. When the transmembrane domain is present, the MBP-GnTII might remain in the inner membrane without reaching the periplasmic space, thus complicating the recovery of the fusion protein.

The DNA coding for MBP in the present invention should especially include a nucleotide sequence coding for an amino acid sequence that is cleaved upon being recognized by a protease with high substrate specificity at the C terminal of the protein (protease recognition site). Examples of such proteases include factor Xa, thrombin, and renin. The protease recognition sequence is preferably an factor Xa recognition sequence, that is, Ile-Glu-gly-Art (SEQ ID NO:3). Such a protease recognition sequence can be introduced to the C terminal of the sugar-binding protein so as to allow the action of the protease after purification of the MBP-GnTII to readily eliminate the MBP.

In a preferred embodiment, the DNA coding for the fusion protein will include a nucleotide sequence coding for a spacer of about 5 to 15 amino acid residues between the sugar-binding protein coding region and the protease recognition site, or between the sugar-binding protein coding region and the GnTII coding region. The spacer is intended to put some distance between the sugar-binding protein and the GnTII so as to minimize the possibility of intramolecular interaction in the fusion protein, and to enhance the affinity for maltose or amylose in the MBP portion of MBP-GnTII fusion proteins.

Based on the well known nucleotide sequence coding foe MBP (such as *E. coli* MBP (Duplay P. et al., J. Biol. Chem., 259:10606-10613 (1984)), the DNA coding for MBP can be cloned by the same well known means as examples for the DNA coding for GnTII, and the protease recognition sequence and/or spacer sequence can be introduced to the C terminal of the MBP in the usual manner. A preferred embodiment of the invention will include the spacer sequence in SEQ ID NO:8.

DNA coding for an *E. coli*-derived MBP can be obtained from commercially available vectors such as pMAL-p2 (including a signal codon) or pMAL-c2 (no signal codon) by New England Biolab.

A suitable restriction enzyme recognition site can be attached in the usual manner to the terminal of the DNA coding for sugar-binding proteins in order to facilitate construction of the chimera DNA coding for the sugar-binding protein-GnTII fusion protein. In cases where a restriction enzyme recognition site is added to the N terminal of GnTII when DNA coding for GnTII is cloned in a vector, the same restriction enzyme recognition site (or another restriction enzyme recognition site producing the same sticky end) should be incorporated in the DNA coding for the sugar-binding protein.

The expression vector of the present invention may be any expression vector in which the chimera DNA coding for the sugar-binding protein-GnTII fusion protein is under the control of a promoter capable of functioning in *E. coli*. The promoter region includes a -35 region and -10 region which are consensus sequences determining the binding site of the RNA polymerase. An induction enzyme promoter region should be used as the system for expressing large amounts of the desired recombinant protein. Examples of such promoter regions include trp promoter, lac promoter, recA promoter, lpp promoter, and tac promoter. These promoter regions also include operators to which repressor proteins bind. The addition of an inducer (such as lactose or IPTG when using a lac promoter) will inhibit the repressor protein binding to the operator, resulting in the expression of large amounts of the recombinant protein under the control of the promoter. The expression vector also contains a consensus Shine-Dalgarno (SD) sequence upstream of the translation start codon. The expression vector also includes a transcription termination signal, that is, a terminator region, down stream of the chimera DNA coding for the sugar-binding protein-GntII fusion protein. Normally used natural or synthetic terminators can be employed as the terminator region. The expression vector of the invention must include an origin of replication capable of autonomous replication in *E. coli* hosts, in addition to the above promoter region and terminator region. Examples of such origins of replication include ColElori and M13ori.

The expression vector of the present invention should also include a selection marker for selection of transformants. Resistance gene against various antibiotics such as tetracycline, ampicillin, and kanamycin can be used as the selection marker. When the *E. coli* host is an auxotrophic mutant, wild type genes complementing the auxotrophic properties can be used as the selection marker.

The transformants of the invention can be prepared by transforming the *E. coli* host with the expression vector of the invention. The host cell line is not particularly limited. Examples include commercially available strain such as XL1-Blue, BL-21, JM107, TB1, JM109, C600, DH5α and HB101.

The expression vector can be introduced into the host cells using a conventionally known method. Examples include the method of Cohen et al (calcium chloride method) (Proc. Natl. Acad. Sci. USA, 69:2110 (1972)), the protoplast method (Mol. Gen. Genet., 168:111 (1979)), the competent cell method (J. Mol Biol., 56:209 (1971)), and electroporation.

The sugar-binding protein-GnTII fusion protein can be obtained by recovering sugar-binding protein-GnTII fusion protein from culture that has been obtained upon cultivation, in suitable media, transformants expressing the expression vector which includes the chimera DNA coding for the sugar-binding protein-GnTII fusion protein.

The media should contain carbohydrates such as glucose, fructose, glycerol, or starch as carbon sources. Inorganic or organic nitrogen sources should also be included (such as ammonium sulfate, ammonium chloride, casein hydrolysates, yeast extracts, polypeptone, bactotryptone, and beef extract). Such carbon and nitrogen sources need not be used in pure form. Those of low purity are advantageous because they contain an abundance of inorganic nutrients or trace amounts of growth factor. Other nutrient sources (such as inorganic salts (for example, sodium diphosphate or potassium diphosphate, dibasic potassium phosphate, magnesium chloride, magnesium sulfate, and calcium chloride), vitamins (such as Vitamin B1), antibiotics (such as ampicillin and kanamycin)) may also be added as desired to the medium.

The transformants are usually cultivated for 1 to 150 hours at a temperature of 18 to 40°C, and preferably 20 to 35°C, at a pH of 5.5 to 8.5, and preferably 6 to 8, but these can be modified as needed depending on culture conditions and the scale of the culture.

To avoid slowing down the growth rate during the production process of the target protein when the cultivation is managed in large tanks, the medium inoculated with a small amount of cells should be cultivated for 1 to 24 hours, and the resulting culture should then be inoculated into the large tanks.

When the expression of the sugar-binding protein-GnTII fusion protein is controlled by an induction protein gene promoter system, the inducer may be added after the start of cultivation, but is preferably added during the initial logarithmic growth phase. Bacterial cell growth can be monitored by measuring the optical density of the culture broth at 660 nm. When, for example, a lac promoter or tac promoter is used, isopropylthio-β-D-galactoside (IPTG) can be added as the inducer in a concentration of between 0.1 and 1.0 mM, for example, when the optical density reaches 0.4 to 0.6 at 660 nm. The period in which the inducer is added and the rate at which it is added can be modified as needed depending on the culture conditions, the scale of the culture, the type of inducer, and the like.

In the method of the invention, the sugar-binding protein-GnTII fusion protein can be purified in a single step by treating fractions containing the fusion protein by affinity chromatography using an insoluble carrier bound various sugar residues as ligand specifically binding to the sugar-binding protein.

For example, when the sugar-binding protein is MBP and the DNA coding for the MBP lacks signal peptide region, the MBP-GnTII fusion protein is localized in the cytoplasm. In such cases, therefore, after cultivation the cultured cells can be recovered by filtration or centrifugation, the cells can be ruptured by lysozyme and surfactant treatment or ultrasonication, and the resulting cell-free extract can apply to affinity chromatography.

When the DNA coding for MBP which have a signal peptide region, on the other hand, it is very likely that the expressed MBP-GnTII fusion protein will be secreted and will accumulate in the periplasmic space. As such, in these cases, the MBP-GnTII fusion protein is extracted from spheroplasts prepared from cultivated cell by lysozyme treatment, the spheroplasts can be removed by filtration or centrifugation, and the resulting supernatant can then apply to affinity chromatography.

In the present invention, fractions containing sugar-binding protein-GnTII fusion protein should be purified as described above in the presence of divalent metal ions. Specifically, the cells are harvested from the culture in the presence of divalent metal ions after cultivation, and the cells are ruptured or the like to obtain fractions containing sugar-binding protein-GnTII fusion protein.

Examples of divalent metal ions include manganese ions (Mn²⁺) and magnesium ions (Mg²⁺), but manganese ions are preferred. Manganese ions can be used in the form of manganese salts such as manganese chloride, manganese sulfate, manganese nitrate, or manganese bromide.

Amylose resin comprising amylose immobilized on agarose beads (such as amylose resin columns by New England Biolabs) can be used as the adsorbent for affinity chromatography in the case of MBP, for example, but other ligands for MBP and other insoluble matrices (such as cellulose, dextran, and synthetic polymers) may also be used.

The adsorbent is added to the fractions containing the sugar-binding protein-GnTII fusion protein prepared according to the above and the mixture is agitated for a suitable period of time. The mixture is then filtered to separate the adsorbent, and the adsorbent is washed. A suitable concentration of eluate containing a sugar (such as maltose in the case of MBP) to inhibit binding between the adsorbent and the sugar-binding protein is furthermore added and mixed for a suitable period of time, and the mixture is then filtered, so that the purified sugar-binding protein-GnTII is obtained in the filtrate. Alternatively, it can be obtained by packing a column with adsorbent, applying fractions containing sugar-binding protein-GnTII fusion protein onto the column, washing the column with a suitable buffer, and then applying a suitable concentration of eluate to elute the sugar-binding protein-GnTII adsorbed to the column.

MBP-GnTII fusion protein of the present invention thus obtained has GnTII activity and can be used as an enzyme.

### Preparation of GnTII

As described above, in a preferred embodiment of the invention, the sugar-binding protein-GnTII fusion protein contains an amino acid sequence that is cleaved by a sequence-specific protease at the fused site, thus allowing the protease to act on the sugar-binding protein-GnTII purified in the manner described above, so that the sugar-binding protein portion is eliminated, giving the desired GnTII. The protease is preferably factor Xa. pMAL-p2 or pMAL-c2 (New England Biolabs) containing the factor Xa recognition sequence of SEQ ID NO. 3 can be used in the present invention. The reaction temperature, solution pH and reaction time used when the protease is allowed to act on the sugar-binding protein-GnTII fusion protein can be adjusted as desired according to the type of protease being used, but in the case of factor Xa, the reaction can be carried out for 1 to 25 hours at 4 to 40°C in neutral buffer to cleave the sugar-binding protein and the GnTII. After the completion of the reaction, the above adsorbent is added to the reaction solution and mixed for a suitable period of time to allow just the free sugar-binding protein to be adsorbed to the adsorbent, and the adsorbent can thus be filtered to purify the GnTII alone. Alternatively, the GnTII can be purified by applying the reaction solution onto a column packed with the adsorbent and collecting the fractions that pass through the column.

### Conversion of Glycoprotein Sugar Chains

As used in the present Specification, high mannose type sugar chains refer. to sugar chains with a structure in which only mannose is linked to the mannose residues at the non-reducing terminal of Manα1-6(Manα1-3)Manβ1-4GlcNAcβ1-4GlcNAc-, which is the core structure of N-linked sugar chains. Specific examples include sugar chains with a Manα1-2Manα1-6(Manα1-2Manα1-3)Manα1-6(Manα1-2Manα1-2Manα1-3)Manβ1-4GlcNAcβ1-4GlcNAc- structure, but also include those in which one or more mannose residues are degraded during the intracellular maturation of the sugar chain structure. Structures including α1-3-linked mannose or α1-6-linked mannose and galactose are also known (Clinton E. Ballou et, al., Proc. Natl. Acad. Sci. USA 91:9327 (1994)).

As used in the present Specification, complex type sugar chains refer to sugar chains with a structure in which the basic skeleton is a structure in which N-acetylglucosamine is the only sugar linked to the mannose residues at the non-reducing terminal of Manα1-6(Manα1-3)Manβ1-4GlcNAcβ1-4GlcNAc-, which is the core structure of N-linked sugar chains. Galactose, sialic acid, fucose, xylose and N-acetylglucosamine may also be linked to the basic skeleton. Specific examples include sugar chains with structures such as GlcNAcβ1-2Manα1-6(GlcNAcβ1-2Manα1-3)Manβ2-4GlcNAcβ1-4GlcNAc-, or Galβ1-4GlcNAcβ1-2Manα1-6(Galβ1-4GlcNAcβ1-2Manα1-3)Manβ1-4GlcNAcβ1-4GlcNAc-, or Siaα2-6Galβ1-4GlcNAcβ1-2Manα1-6(Siaα2-6Galβ1-4GlcNAcβ1-2Manα1-3)Manβ1-4GlcNAcβ1-4GlcNAc-. The N-acetylglucosamine need not necessarily be linked by the β1-2 linkage to the non-reducing end of the core structure Manα1-6(Manα1-3)Manβ1-4GlcNAcβ1-4GlcNAc- of the N-linked sugar chain, but may also be linked, for example, by β1-3, β1-4, β1-6, α1-2, α1-3, α1-4, and α1-6 linkages.

As used in the present Specification, hybrid-type sugar chains refer to sugar chains with a structure in which the basic skeleton is a structure in which mannose is the sugar linked to the α1-6 mannose residues at the non-reducing terminal of Manα1-6(Manα1-3)Manβ1-4GlcNAcβ1-4GlcNAc-, which is the core structure of N-linked sugar chains, and N-acetylglucosamine is the sugar linked to the α1-3 mannose residues. Galactose, sialic acid, fucose, xylose and N-acetylglucosamine may also be linked to the basic skeleton. Specific examples include sugar chains with structures such as Manα1-6(Manα1-3)Manα1-6(Siaα2-3Galβ1-4GlcNAcβ1-2Manα1-3)(Xylβ1-2)Manβ1-4GlcNAcβ1-4(Fucα1-3)GlcNAc.

Examples of glycoproteins which have been expressed by genetic engineering and which have high mannose type sugar chains with 2 to 9 mannose residues include glycoproteins obtained by genetic engineering using hosts such as CHO or other mammal cells, yeasts, insect cells, molds, chicken cells or eggs, algae, plant cells or other eukaryote cells, or mammal, insect, or plant hosts.

Hosts for the production of recombinant proteins with modified sugar chain structures have been improved using recent genetic engineering techniques. It is reported that the recombinant yeast cell comprising the *Aspergillus saitoi* α1,2-mannosidase gene incorporated into triple mutant (och1, mnn1, mnn4) *Saccharomyces cerevisiae* produce glycoprotein bound high mannose type sugar chain containing 5 mannose residues, in which the glycoprotein sugar chain structure is an optimal substrate for β1,2-N-acetylglucosaminyltransferase I (Chiba Y., et al., J. Biol. Chem., 273:26298-26304 (1988)). The introduction and expression of a useful exogenous protein gene in this recombinant yeast host results in the recombinant glycoprotein bound high mannose type sugar chain structure with five mannose residues, and the action of β1,2-N-acetylglucosaminyl transferase I can result in the conversion to a glycoprotein having a GlcNAcβ1-2Manα1-3Manβ1-4 moiety. Alternatively, a useful exogenous gene can be introduced into triple mutant (och1, mnn1, mnn4) *Saccharomyces cerevisiae* yeast and expressed. The subsequent action of *Aspergillus saitoi* α1,2-mannosidase or mannosidase I and followed by the action of β1,2-N-acetylglucosaminyl transferase I can result in the conversion to a glycoprotein with a GlcNAcβ1-2Manα1-3Manβ1-4 moiety.

Glycoproteins with a sugar chain structure consisting of 5 to 8 mannose residues have been obtained with another triple mutant (och1, mnn1, alg3) *Saccharomyces cerevisiae* yeast (Nakanishi-Shindo Y., J. Biol. Chem., 268:26338-26345 (1993)). The action of *Aspergillus saitoi* α1,2-mannosidase or mannosidase I, followed by the action of β1, 2-N-acetylglucosaminyltransferase I, can result in the conversion to a glycoprotein with a GlcNAcβ1-2Manα1-3Manβ1-4 moiety.

*Arabidopsis thaliana* plant cells with a mutation in the β1,2-N-acetylglucosaminyltransferase I gene have been discovered (von Schaewen A. et al., Plant Physiol., 102:1109-1113(1993)). This mutant plant gives a glycoprotein with a high mannose type sugar chain containing 5 mannose residues. Manα1-6(Manα1-3)Manα1-6(Manα1-3)Manβ1-4 with a sugar chain structure containing of 5 mannose residues is a more desirable substrate for β1,2-N-acetyl glucosaminyltransferase I. Application of the cDNA of β1,2-N-acetyl glucosaminyltransferase I cloned from plants to tobacco plants for antisense method or cosupression method gave plants with reduced β1,2-N-acetylglucosaminyltransferase activity (Wenderoth I. and von Schaewen A., Plant Physiol., 123:1097-1108(2000)). The incorporation and expression of a useful exogenous gene in such recombinant plant hosts can result in a high mannose type sugar chain structure containing 5 mannose residues, and the in vitro action of β1, 2-N-acetylglucosaminyltransferase I can result in the conversion to glycoproteins with a GlcNAcβ1-2Manα1-3Manβ1-4 moiety.

Regardless of the type of protein, all glycoproteins are encompassed, such as RNaseB.

In step (1) of item (30) the sugar chain structure can be converted from, for example, a hybrid-type sugar chain (item (30)) or a high mannose type sugar chain (item (30)) (see Figure 9) to the Manα1-6(Manα1-3)Manα1-6(Manα1-3)Manβ1-4GlcNAcβ1-4GlcNAc structure, and α1,2-mannosidase is the preferred glycosidase to use in these steps. α1,2-mannosidase is an enzyme that hydrolyzes mannose residues linked by the α1,2 linkage. Mannosidase I and Man₉-mannosidase are also included in α1,2-mannosidase in the present invention. When the outer chain of sugar chain on the glycoprotein intended to convert its sugar chain includes α1-3-linked mannose or α1-6-linked mannose, α-mannosidase can also be used. Furthermore, at least one selected from the group consisting of xylosidase, fucosidase, N-acetylglucosaminidase, galactosidase and sialidase can also be used as the glycosidase when xylose, fucose, N-acetylglucosamine, galactose or sialic acid is included in sugar chains on the glycoprotein intended to convert its sugar chain.

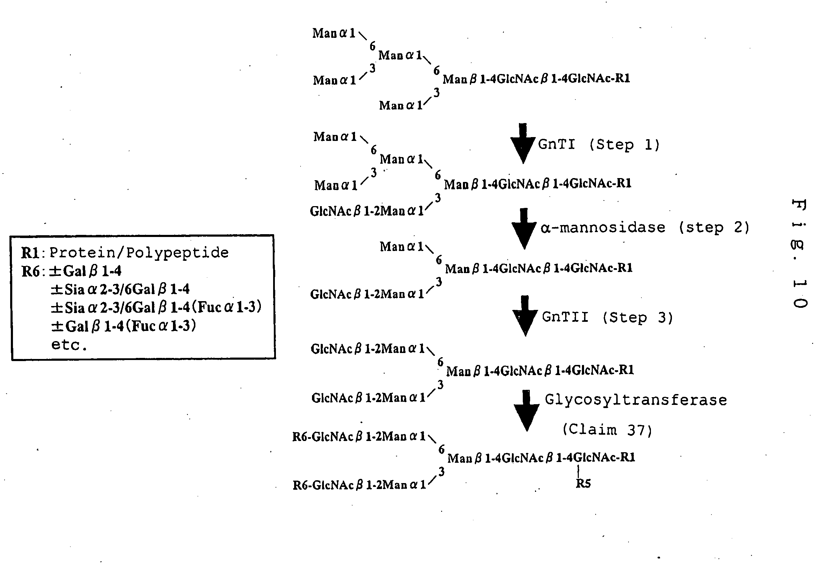

In step (3) of item (30) or step (2) of item (41), the sugar chain structure can be converted from, for example, a Manα1-6(Manα1-3)Manα1-6(GlcNAcβ1-2Manα1-3)Manβ1-4GlcNAcβ1-4GlcNAc structure to a Manα1-6(GlcNAcβ1-2Manα1-3)Manβ14GlcNAcβ1-4GlcNAc structure (see Figures 9 and 10), and the α-mannosidase used in this case is an enzyme that hydrolyzes mannose linked by the α1,2-linkage, α1,3-linkage, α1,4-linkage, or α1,6-linkage. α-mannosidase II is an enzyme that hydrolyzes mannose linked by the α1,3-linkage or α1,6-linkage, but its substrate specificity is more stringent, making it a more desirable enzyme for this process in terms of catalyzing the reaction Manα1-6(Manα13) 3)Manα1-6(GlcNAcβ1-2Manα1-3)Manβ1-4GlcNAcβ1-4GlcNAc □ Manα1-6(GlcNAcβ1-2Manα1-3)Manβ1-4GlcNAcβ1-4GlcNAc, without deleting the mannose up to GlcNAcβ1-2Manα1-3)Manβ1-4GlcNAcβ1-4GlcNAc (J. Biol. Chem. 266: 16876 (1991)).

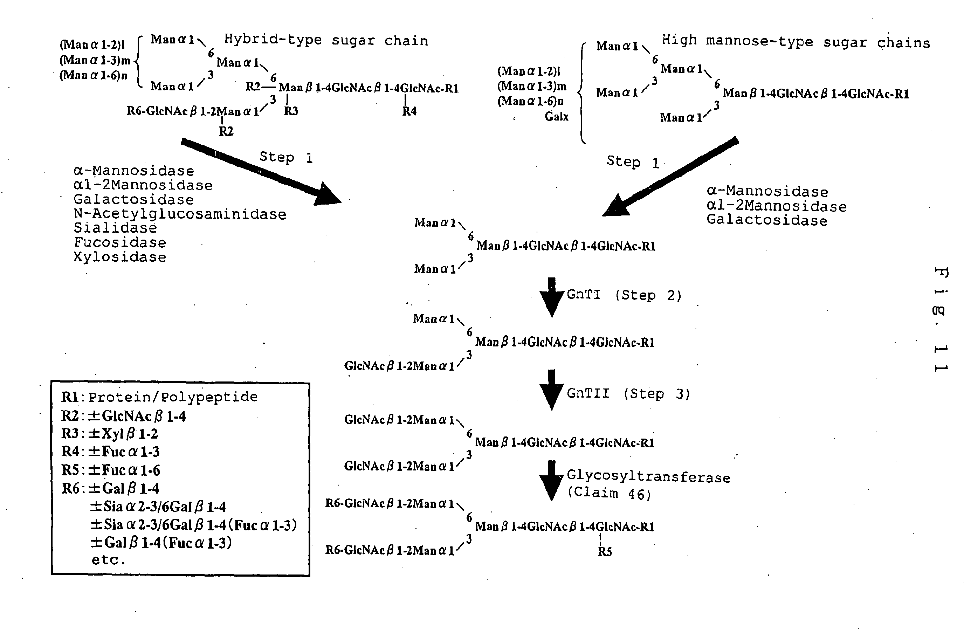

In step (1) of item (22), the sugar chain structure can be converted, for example, from a hybrid-type sugar chain or high mannose type sugar chain (see Figure 11) to a Manα1-6(Manα1-3) Manβ1-4GlcNAcβ1-4GlcNAc structure, and the glycosidase used in this step is preferably α-mannosidase. α-mannosidase is an enzyme that hydrolyzes mannose linked by the α1,2-linkage, α1,3-linkage, α1,4-linkage, or α1,6-linkage. Also, when xylose, fucose, N-acetylglucosamine, galactose or sialic acid is added to the converted glycoprotein sugar chain, then xylosidase, fucosidase, N-acetylglucosaminidase, galactosidase or sialidase, which can hydrolyze the above can also be used with the α-mannosidase.

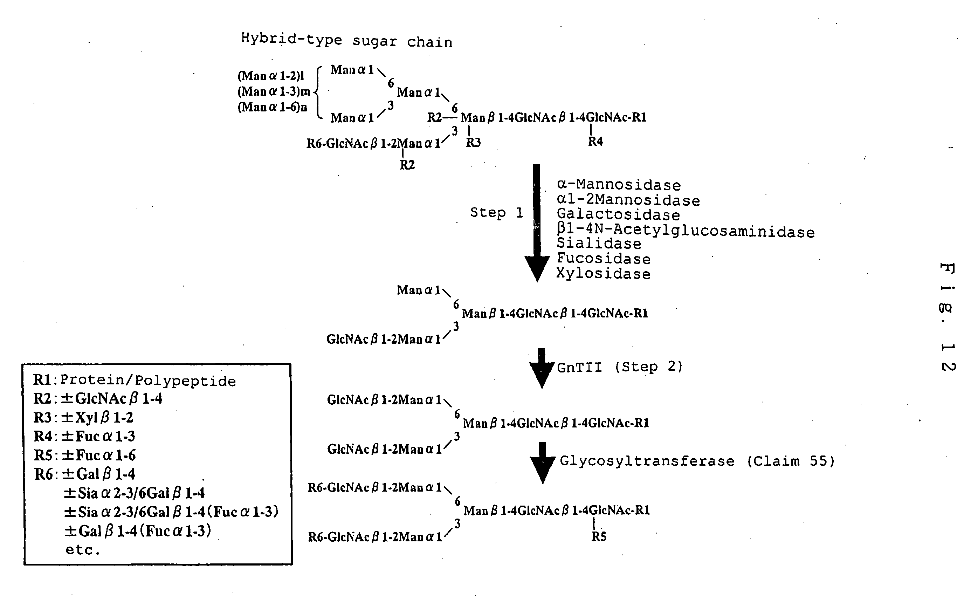

In step (1) of item (49), the sugar chain structure can be changed from, for example, a hybrid-type glycoprotein (see Figure 12) to a Manα1-6(GlcNAcβ1-2Manα1-3)Manβ14GlcNAcβ1-4GlcNAc structure, but the glycosidase used in this step is preferably α1,2-mannosidase. α1,2-mannosidase is an enzyme that hydrolyzes mannose residues linked by the α1,2-linkage. Mannosidase I and Man₉-mannosidase are also included in α1,2-mannosidase in the present invention. When the outer chain of the converted glycoprotein sugar chain includes α1-3-linked mannose or α1-6-linked mannose, α-mannosidase can also be used. Furthermore, when xylose, fucose, N-acetyl glucosamine, galactose or sialic acid is included in sugar chains on the glycoprotein intended to convert its sugar chain, then at least one of selected from the group consisting xylosidase, fucosidase, N-acetylglucosaminidase, galactosidase or sialidase can also be used with the α-mannosidase. However, the β1,2-N-acetylglucosaminidase is not desirable'because it will inhibit the formation of Manα1-6(GlcNAcβ1-2Manα1-3) Manβ1-4GlcNAcβ1-4GlcNAc, which is the target structure.

β1,2-N-acetylglucosaminyltransferase I (GnTI) is an enzyme that has the action of transferring GlcNAc via β1-2 linkages to the acceptor Manα1-6 (Manα1-3)Manα1-6 (Manα13)Manβ1-4GlcNAcβ1-4GlcNAc-R in the presence of UDP-GlcNAc as the sugar donor, so as to produce Manα1-6(Manα1-3)Manα1-6(GlcNAcβ1-2Manα1-3)Manβ1-4GlcNAcβ1-4GlcNAc-R (where R is asparagine residue, peptide, protein, or a low- or high-molecular weight compound which does not inhibit the activity of other glycosyltransferases). It also transfers GlcNAc via β1-2 linkages to Manα1-6(Manα1-3)Manβ1-4GlcNAcβ1-4GlcNAc-R or Manα1-6(Manα1-2Manα1-3)Manα1-6(Manα1-3)Manβ1-4GlcNAcβ1-4GlcNAc-R.

β1,2-N-acetylglucosaminyltransferase II (GnTII) is an enzyme that has the action of transferring GlcNAc by means of β1-2 linkages to the acceptor Manα1-6(GlcNAcβ12Manα1-3)Manβ1-4GlcNAcβ1-4GlcNAc-R using at least UDP-GlcNAc as the sugar donor, so as to produce GlcNAcβ12Manα1-6(GlcNAcβ1-2Manα1-3)Manβ1-4GlcNAcβ1-4GlcNAc-R (where R is asparagine residue, peptide, protein, or a low- or high-molecular weight compound which does not inhibit the activity of other glycosyltransferases).

Glycoproteins with a converted sugar chain structure are easier to separate from the reaction system when at least one of the α1,2-mannosidase, β1,2-N-acetyl glucosaminyltransferase I, α-mannosidase, or β1,2-N-acetylglucosaminyltransferase II used in the invention is an immobilized enzyme.

In step (1) of item (30), glycoproteins having high mannose type sugar chains or hybrid-type sugar chains can be hydrolyzed under the following conditions. It will be apparent to those having ordinary skill in the art that glycoproteins having other sugar chains can also be hydrolyzed under appropriate conditions with reference to these conditions. It is shown in figure 9 that the sugar chain structure can be converted from high mannose type sugar chains or the hybrid-type sugar chains to Manα16(Manα1-3)Manα1-6(Manα1-3)Manβ1-4GlcNAcα1-4GlcNAc, for example. In this step, the conversion can be carried out by allowing a suitable amount of a glycosidase such as α1,2-mannosidase to act for about 1 to 72 hours on a glycoprotein containing high mannose type sugar chains at a temperature from room temperature to about 40°C in buffer with a pH of about 5 to 7 such as acetate buffer (pH 5.0) or MES buffer (pH 6.5). Alternatively, endoplasmic reticulum mannosidase can be allowed to act first prior to the action of the α1,2-mannosidase. α-mannosidase or galactosidase can also be allowed to act first in cases of the sugar chais including α1-3 mannose residue, α1-6 mannose residue, or galactose residue.

In this step, the conversion can also be carried out by allowing a suitable amount of a α1,2-mannosidase to act for about 1 to 72 hours on a glycoprotein containing hybrid-type sugar chains at a temperature from room temperature to about 40°C in buffer with a pH of about 5 to 7 such as acetate buffer (pH 5.0) or MES buffer (pH 6.5). Alternatively, endoplasmic reticulum mannosidase can be allowed to act first prior to the action of the α1,2-mannosidase. α-mannosidase, sialidase, galactosidase, xylosidase, fucosidase N-acetyl glucosaminidase can also be allowed to act first in cases of the sugar chains including α1-3 mannose residues, α1-6 mannose residue, sialic acid residue, galactose residue, xylose residue, fucose residue or N-acetylglucosamine residue.

The target product can be purified by dialysis after the completion of the reaction, but the target material is more readily purified in the case of using an immobilized enzyme such as immobilized α1,2-mannosidase.

Step (2) of item (30) can be carried out by allowing a suitable amount of β1,2-N-acetylglucosaminyltransferase I, in the presence of an equimolar or excess amount of UDP-GlcNAc, to act for about 1 to 72 hours at room temperature to about 40°C on the glycoprotein obtained in step (1), in buffer with a pH of about 5 to 7 such as acetate buffer (pH 5.0) or MES buffer (pH 6.5). It is required for glycosylation by β1, 2-N-acetylglucosaminyltransferase I that no sugar residue is linked to the non-reducing end of the Manα1-3Manβ1-4 branch of the sugar chain on the glycoprotein. The sugar chain structure Manα1-6(Manα1-3)Manα1-6(Manα1-3)Manβ1-4 containing of 5 mannose residues is an ideal substrate for β1,2-N-acetylglucosaminyltransferase I. However, the sugar chain structure Manα1-6(Manα1-3)Manβ1-4 containing of 3 mannose residues found in step (2) of item (22) is a more desirable substrate for β1,2-N-acetylglucosaminyltransferase I. The 6-mannose residue containing sugar chain structure Manα1-6(Manα1-2Manα1-3)Manα1-6(Manα1-3)Manβ1-4 is also a substrate for β1,2-N-acetylglucosaminyltransferase I. The sugar chain structure Manα1-6Manα1-6(Manα1-3)Manβ1-4 and Manα1-3Manα1-6(Manα1-3)Manβ1-4 can also serve as substrates for β1, 2-N-acetylglucosaminyltransferase I. After the mannosidase treatment process in (1) above, it is no matter that the high mannose type sugar chains of the glycoproteins obtained have 3, 4, or 6 mannosidase residues. The target glycoprotein can be purified by dialysis.

The target material can also be more readily purified with the use of immobilized β1,2-N-acetyl glucosaminyltransferase I.

In step (3) of item (22), Manα1-6(Manα1-3)Manα1-6(GlcNAcβ1-2Manα1-3)Manβ1-4GlcNAcβ1-4GlcNAc (see Figure 9), for example, can be converted to the GnTII substrate Manα1-6(GlcNAcβ1-2Manα1-3)Manβ1-4GlcNAcβ1-4GlcNAc. This step can be carried out by allowing a suitable amount of α-mannosidase to act on the glycoprotein obtained in step 2, in a buffer with a pH of about 5 to 8, acetate buffer (pH 5.0), MES buffer (pH 6.5), and Tris-HCl buffer (pH 7.5). Examples of commercially available α-mannosidase include α-mannosidase derived from jack beans and α-mannosidase derived from almonds, and purified α-mannosidase or α-mannosidase II extracts from other organism sources can also be used.

Human mannosidase II isozymes or homologue genes which have been reported may also be used. Bacterial cell-derived mannosidase that hydrolyse α-1,2/3 linkages and α-1,6 linkages is commercially available (such as from New England Biolab). These may be used in combination.

Although the target glycoprotein can be purified by dialysis, the target material is more readily purified in the case of immobilized α-mannosidase.

In step (4) of item (30), a suitable amount of β1,2-N-acetylglucosaminyltransferase II, in the presence of an equimolar or excess amount of UDP-GlcNAc, are allowed to act for about 1 to 72 hours at room temperature to about 40°C on the glycoprotein obtained in step 3, in buffer with a pH of about 5 to 7 such as acetate buffer (pH 5.0) or MES buffer (pH 6.5), and the resulting material is purified by dialysis, allowing the GlcNAc to be linked to the glycoprotein via β1,2-linkage. The target material can also be more readily purified in the case of immobilized β1,2-N-acetylglucosaminyltransferase I.

It is also possible to act galactosyltransferase (such as β1,3-galactosyltransferase or β1,4-galactosyltransferase) in the presence of UDP-Gal, sialyltransferase (such as α2,6-sialyltransferase or α2,3-sialyltransferase) in the presence of CMP-Sia, or fucosyltransferase (such as α1,2-fucosyltransferase, α1,3-fucosyltransferase, α1,4-fucosyltransferase, or α1,6-fucosyltransferase) in the presence of GDP-Fuc on the glycoprotein obtained in the manner described above.

The target material is also more readily purified in the case of an immobilized glycosyltransferase. The glycosyltransferase used in the present invention may be produced naturally or by genetically engineering.

Step (1) in item (41) can be carried out in the same manner as step (2) in item (30) except that a recombinant glycoprotein is used instead of the glycoprotein obtained in step (1) of item (30).

Step (2) of item (41) can be carried out in the same manner as step (3) in item (30) using the glycoprotein obtained in step (1) of item (41) instead of the glycoprotein obtained in step (2) of item (30).

Step (3) of item (41) can be carried out in the same manner as step (4) in item (30) using the glycoprotein obtained in step (2) of item (41) instead of the glycoprotein obtained in step (3) of item (30).

It is also possible to act galactosyltransferase (such as β1,3-galactosyltransferase or β1,4-galactosyltransferase) in the presence of UDP-Gal, sialyltransferase (such as α2,6-sialyltransferase or α2,3-sialyltransferase) in the presence of CMP-Sia, or fucosyltransferase (such as α1,2-fucosyltransferase, α1,3-=fucosyltransferase, α1,4-fucosyltransferase, or α1,6-fucosyltransferase) in the presence of GDP-Fuc on the glycoprotein obtained in the manner described above.

The target material is also more readily purified in the case of an immobilized glycosyltransferase. The glycosyltransferase used in the present invention may be produced naturally or by genetically engineering.

In step (1) of item (22), the glycoprotein can be converted, for example, from high mannose type sugar chains or hybrid-type sugar chains to Manα1-3(Manα1-6) Manβ1-4GlcNAcβ1-4GlcNAc (see Figure 11). This step can be carried out by allowing a suitable amount of α-mannosidase to act on the glycoprotein obtained in step 2, in a buffer with a pH of about 5 to 7, such as acetate buffer (pH 5.0) or MES buffer (pH 6.5). Sialidase, galactosidase, xylosidase, fucosidase or N-acetylglucosaminidase can also be allowed to act first in cases of sugar chain on glycoprotein including sialic acid residue, galactose residue, xylose residue, fucose residue or N-acetylglucosamine residue.

The target product can be purified by dialysis after the conclusion of the reaction, but the target material is more readily purified in the case of an immobilized enzyme such as immobilized α-mannosidase.

Step (2) of item (22) can be carried out in the same manner as step (2) in item (30) using the glycoprotein obtained in step (1) of item (22) instead of the glycoprotein obtained in step (1) of item (30).

Step (3) of item (22) can be carried out in the same manner as step (4) in item (30) using the glycoprotein obtained in step (2) of item (22) instead of the glycoprotein obtained in step (3) of item (30).

It is also possible to act galactosyltransferase (such as β1,3-galactosyltransferase or β1,4-galactosyl transferase) in the presence of UDP-Gal, sialyltransferase (such as α2,6-sialyltransferase or α2,3-sialyltransferase) in the presence of CMP-Sia, or fucosyltransferase (such as α1,2-fucosyltransferase, α1,3-fucosyltransferase, α1,4-fucosyltransferase, or α1,6-fucosyltransferase) in the presence of GDP-Fuc on the glycoprotein..sugar chains obtained in the manner described above.

The target material is also more readily purified in the case of an immobilized glycosyltransferase. The glycosyltransferase used in the present invention may be produced naturally or by genetically engineering.

In step (1) of item (49), hybrid-type sugar chains (see Figure 12) on glycoproteins, for example, can be converted to a Manα1-3(GlcNAcβ1-2Manα1-6)Manβ1-4GlcNAcβ1-4GlcNAc structure. This step can be carried out in the same manner as step (1) in item (22) except that only glycoproteins with hybrid-type sugar chains are used in step (1) of item (22), and no β1,2-N-acetylglucosaminidase is used as the glycosidase.

Step (2) of item (49) can be carried out in the same manner as step (3) in item (22) using the glycoprotein obtained in step (1) of item (49) instead of the glycoprotein obtained in step (2) of item (22).

### BEST MODE FOR CARRYING OUT THE INVENTION

The present invention is illustrated in further detail in the following examples, but these are examples.

### Example 1: Obtaining hGnTIIcDNA Fragments by PCR

The oligonucleotides represented in SEQ ID NO. 4 and 5 were synthesized based on the nucleotide sequence for the human β1,2-N-acetylglucosaminyltransferase II (hGnTII) cDNA in SEQ ID NO. 1. The former is the nucleotide sequence 4-27 in the nucleotide sequence in SEQ ID NO. 1, and the latter is the nucleotide sequence 1341-1377 in the nucleotide sequence in SEQ ID NO:1. These oligonucleotides were used as primers in PCR under the following conditions using a Caucasian female placenta chromosomal DNA (Clontech) as template.

{1 µL of 0.1 mg/mL Caucasian female placenta chromosomal gene, 1 µL each of 10 pmole/µL primer, 0.5 µL Taq. DNA Polymerase (Takara PCR Kit, by Takara Shuzo), 5 µL of 10-fold concentrated PCR buffer (Takara PCR Kit, by Takara Shuzo), 4 µL dNTP (Takara PCR Kit, by Takara Shuzo), 37.5 µL sterilized water, total reaction solution was 50 µL, reaction temperature (time): denaturation 94°C (2 min), annealing 50°C (2 min), elongation 72°C (1 min); number of cycles: 30.}

After the PCR reaction, the reaction solution was applied to agarose gel electrophoresis to check the DNA fragment (PCR product). The DNA fragment was ligated to the pGEM-Teasy (Promega) vector with T4 DNA ligase according to TA cloning method, *E. coli* JM109 was transformed, and transformants were selected on LB plate media containing 50 µg/mL ampicillin, IPTG, and X-gal.

Plasmids with the DNA fragments were prepared from the *E. coli,* and the DNA fragment nucleotides were sequenced in the usual manner, revealing that the sequence was consistent with the nucleotide sequence 4-1377 in the nucleotide sequence of SEQ ID NO:1. It was thus confirmed that the desired DNA coding for hGnTII had been obtained.

### Example 2: Construction of Expression Vector pMGNT-II

The oligonucleotides in SEQ ID NOs:6 and 7 were synthesized based on the nucleotide sequence for β1,2-N-acetyl glucosaminyltransferase II (hGnTII) cDNA in SEQ ID NO. 1 in order to obtain DNA coding for the GnTII lacking the transmembrane domain. In the former, a BamHI recognition site was ligated to the 5' terminal of the nucleotide sequence 85-105 in the nucleotide sequence of SEQ ID NO:1, and in the latter, a HindIII recognition site was ligated to the 3' terminal of the nucleotide sequence 1334-1359 in the nucleotide sequence of SEQ ID NO. 1. These oligonucleotides were used as primers for PCR under the following conditions using the hGnTII cDNA on the pGEM-Teasy obtained in Example 1 as template.

{1 µL of 0.1 mg/mL hGnTII cDNA obtained in Example 1, 1 µL each of 10 pmole/µL primer, 0.5 µL Taq. DNA Polymerase (Takara PCR Kit, by Takara Shuzo), 5 µL of 10-fold concentrated PCR buffer (Takara PCR Kit, by Takara Shuzo), 4 µL dNTP (Takara PCR Kit, by Takara Shuzo), 37.5 µL sterilized water, total reaction soulution is 50 µL, reaction temperature (time): denaturation 94°C (2 min), annealing 50°C (2 min), elongation 72°C (1 min); number of cycles: 30.}

The PCR products were digested with the BamHI and HindIII restriction enzymes, and approximately 1.2 kb fragments were recovered from electrophoresed agarose gel. Meanwhile, the pMAL-c2 plasmid (New England Biolabs) was digested with the BamHI and HindIII restriction enzymes, and approximately 6.6 kb fragments were recovered from electrophoresed agarose gel. The two fragments were ligated using T4 DNA ligase, giving the novel plasmid pMGNT-II.

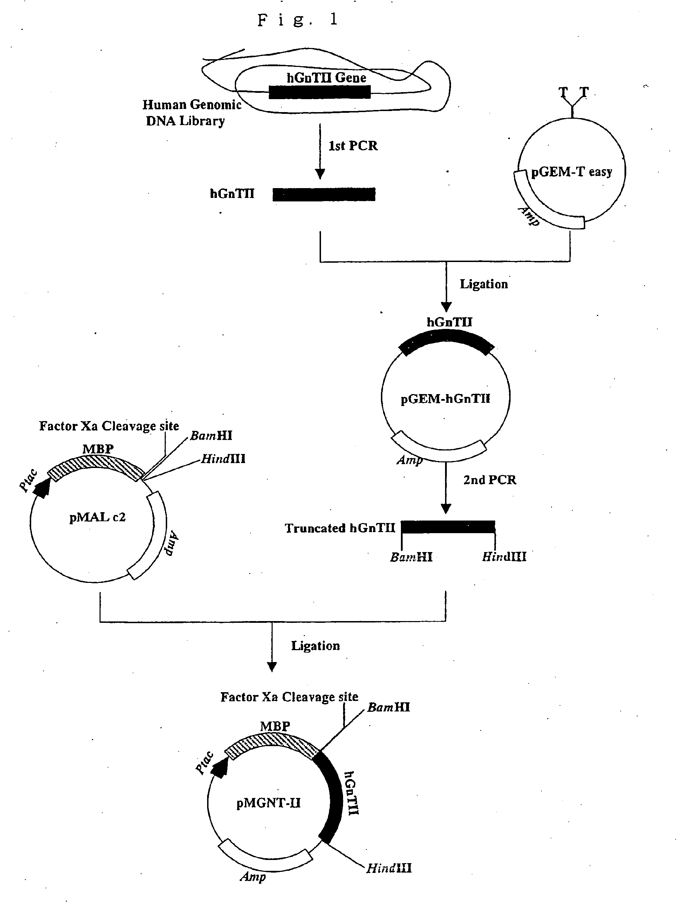

The above procedure is summarized in Figure 1.

### Example 3: Preparation of MBP-hGnTII Fusion Protein Using Recombinant E. coli

*E. coli* DH5α was transformed in the usual manner with the pMGNT-II expression vector obtained in Example 2. Two test tubes filled with 5 mL of LB medium containing 50 µg/mL ampicillin were inoculated with 1 platinum loop of the resulting recombinant *E. coli* for shaking culture overnight at 37°C. 10 mL of the resulting culture was used to inoculate culture flasks filled with 1 L of the above medium. When the optical density of the culture at 610 nm reached 0.5 during shaking culture at 37°C, IPTG was added to a final concentration of 1 mM, and the cultivation was continued for another 4 hours at 37°C.

The cells were harvested by centrifugation and resuspended in 23 mL of 100 mM MES buffer (pH 6.5, containing 20 mM MnCl₂, 50 mM NaCl, and 1 mM 2-mercaptoethanol). The suspension was treated with a sonicator and centrifuged, giving supernatant in the form of a crude enzyme solution. The crude enzyme solution was allowed to flow through an amylose resin (New England Biolabs) column (5 mL) which had been previously equilibrated with the above buffer, thereby allowing the MBP-hGnTII fusion protein to be adsorbed. The column was washed with the above buffer, and the adsorbed fusion protein was eluted with the above buffer containing 10 mM maltose. The eluted fractions were collected, and the resulting fusion protein was dialyzed against the above buffer, giving a fusion protein solution(MBP-hGnTII).

### Example 4: Assay of GnTII Activity

A reaction was carried out for 3 hours at 37°C between 23 µL of the fusion protein (MBP-hGnTII) obtained in Example 3, 1 µL of 308 mM uridine-5'-diphospho-N-acetylglucosamine (UDP-GlcNAc), and reaction solution containing 1 µL of 10 µM pyridylaminated oligosaccharide (Takara Shuzo) represented by Formula 2 below.

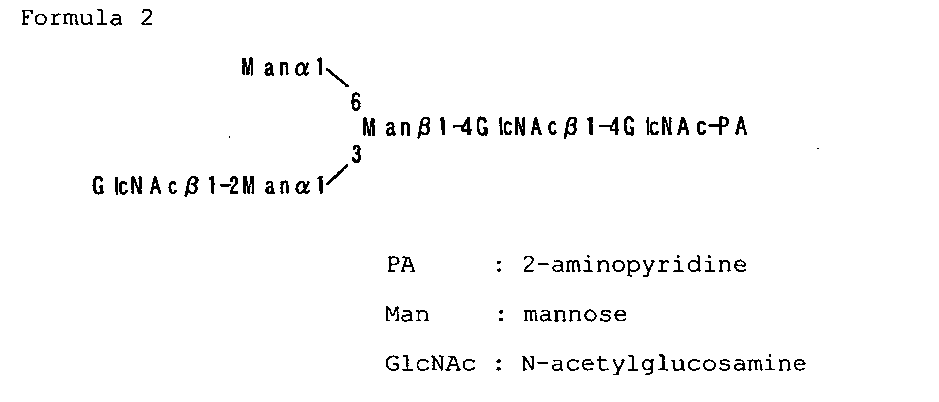

After the reaction, the reaction was stopped by 2 minutes of incubation in boiling water. Analysis of the reaction solution by HPLC revealed a product (Formula 3) to which N-acetylglucosamine had been transferred.

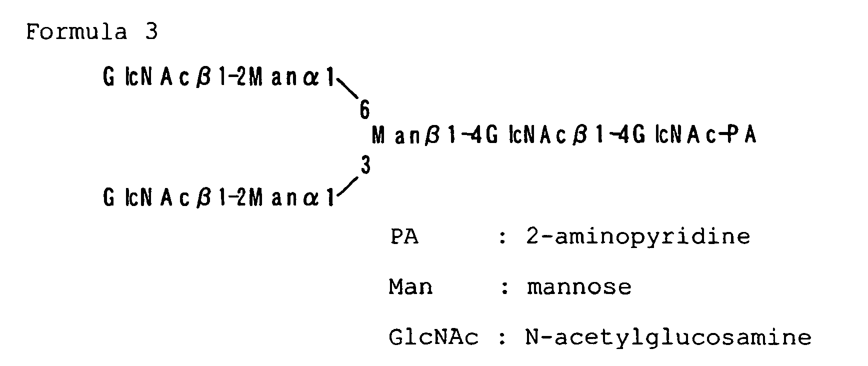

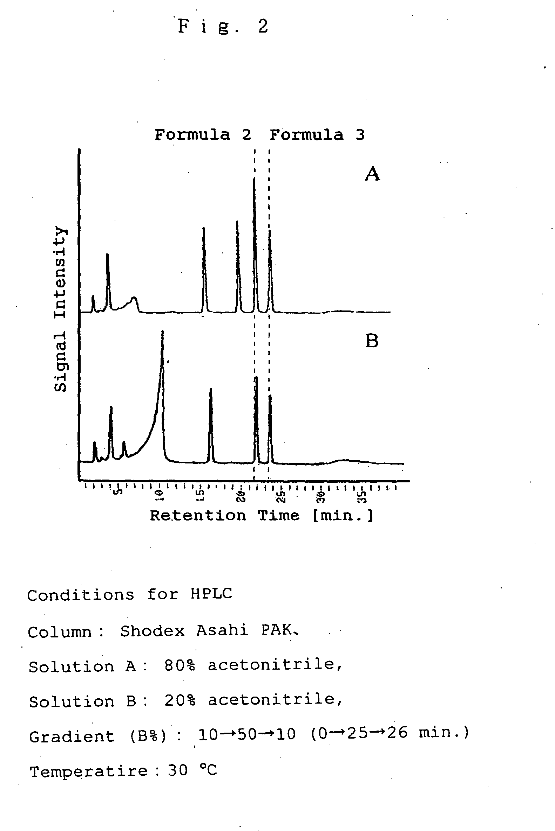

The results are given in Figure 2. Figure 2A gives the results of analysis for the standard pyridylaminated oligosaccharides (Takara Shuzo) represented by Formulas 2 and 3, and Figure 2B gives the results from 3 hours of reaction using the pyridylaminated oligosaccharide represented by Formula 2 as the substrate. The peak for the pyridylaminated oligosaccharide represented by Formula 3 in Figure 2B was produced by MBP-hGnTII.

The above results confirmed that the MBP-hGnTII obtained by the method of the invention had GnTII enzyme activity.

### Example 5: Study on hGnTII Substrate Specificity

Reaction was carried out overnight at 37°C in 50 µL of reaction solution containing the MBP-hGnTII obtained in Example 3 (23 µL), 308 mM UDP-GlcNAc (1 µL), and 1 µL of 10 µM pyridylaminated oligosaccharides represented by Formulas 2 and 4-8. After the reaction, the reaction was stopped by 2 minutes of incubation in boiling water. Analysis of the reaction solution by HPLC in the same manner as in Example 4 revealed activity only for the pyridylaminated oligosaccharide of Formula 6 other than the pyridylaminated oligosaccharide of Formula 2 (Table 1). The above results confirmed that the MBP-hGnTII obtained by the method of the invention had the same substrate specificity as the natural one.

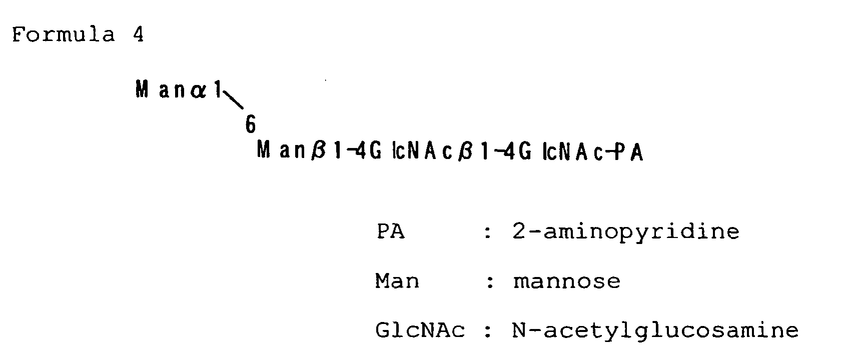

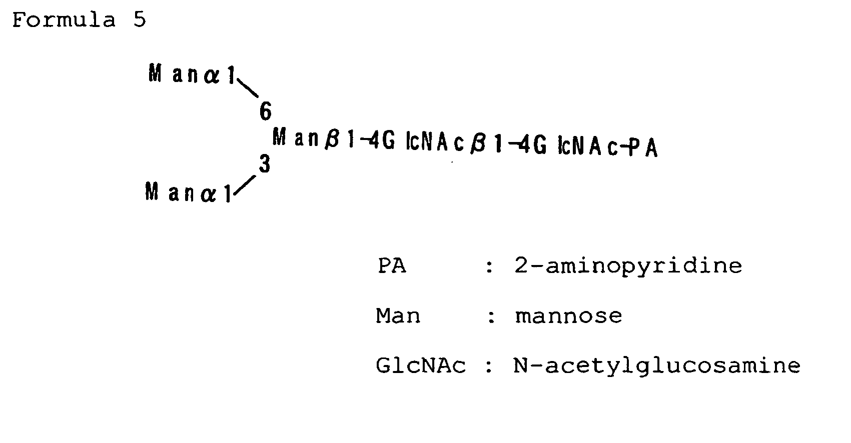

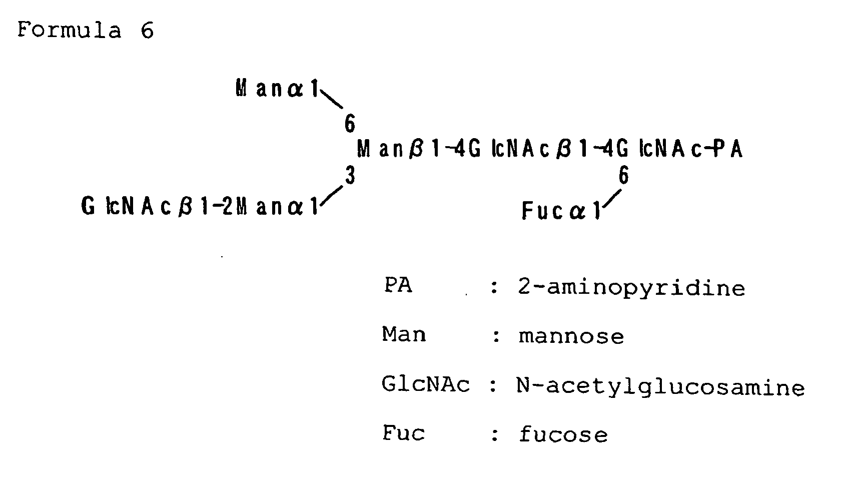

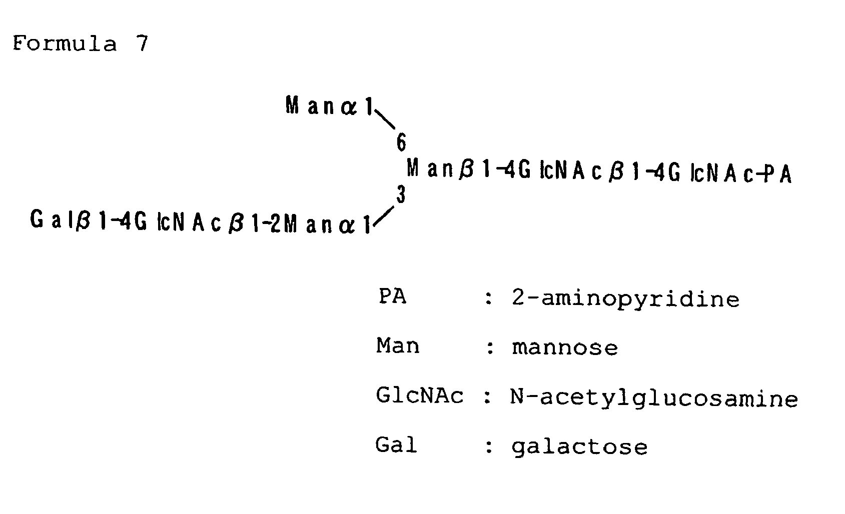

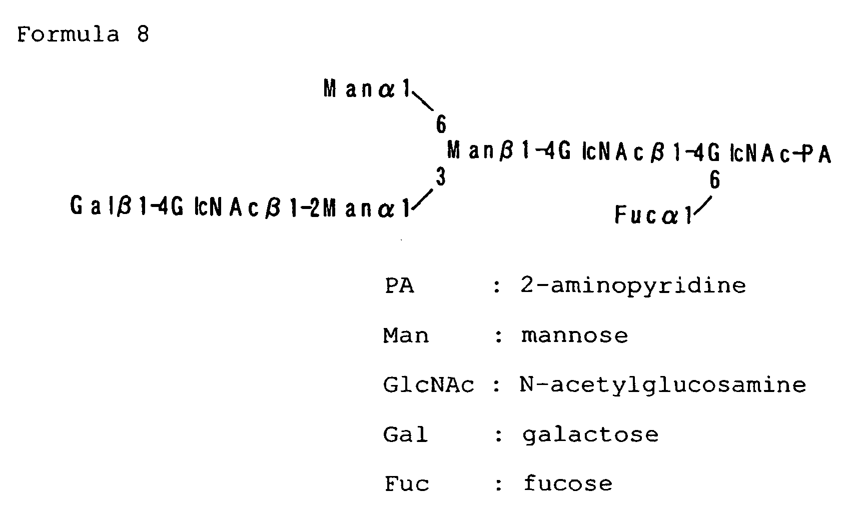

**Table 1**

| Sugar Chain Substrate | GnTII activity |
|---|---|
| Formula 2 | + |
| Formula 4 | - |
| Formula 5 | - |
| Formula 6 | + |
| Formula 7 | - |
| Formula 8 | - |

### Example 6: Study on Optimal Reaction Temperature for MBP-hGnTII

Reaction was carried out for 3 hours at temperatures from 0 to 70°C in 50 µL of reaction solution containing the MBP-hGnTII obtained in Example 3 (23 µL), 308 mM UDP-GlcNAc (1 µL), and 1 µL of 10 µM pyridylaminated oligosaccharide represented by Formula 2. After the reaction, the reaction was stopped by 2 minutes of incubation in boiling water. The reaction solutions were analyzed by HPLC in the same manner as in Example 4 to assay GnTII activity and the optimal temperature reaction for the MBP-hGnTII.

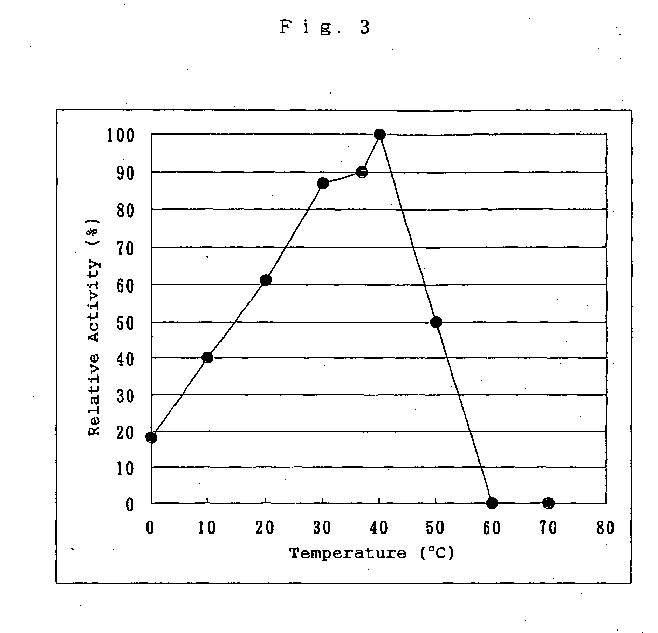

The optimal reaction temperature was found to be between 30 and 40°C (Figure 3).

### Example 7: Study on Thermal Stability of MBP-hGnTII

The MBP-hGnTII obtained in Example 3 (23 µL) was incubated for 4 hours at temperatures between 0 and 70°C, and was then cooled on ice for 5 minutes. 308 mM UDP-GlcNAc (1 µl) and 1 µl of 10 µM pyridylaminated oligosaccharide represented by Formula 2 were added, and a reaction was carried out for 5 hours at 37°C.

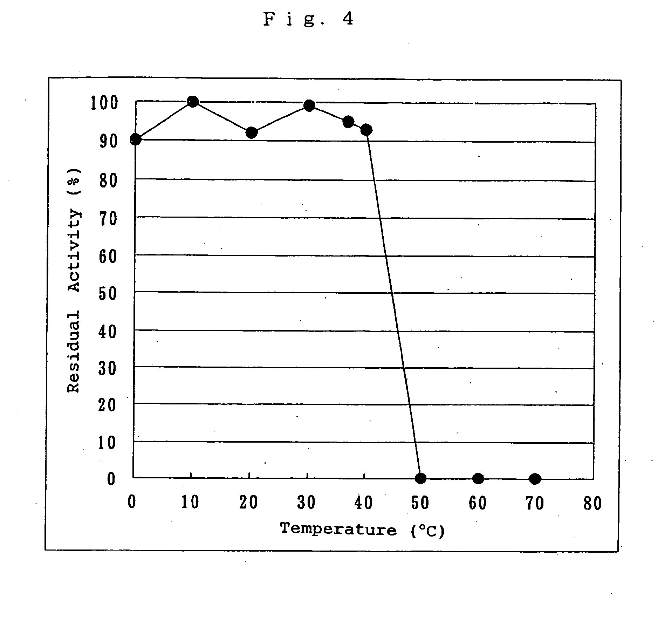

After the reaction, the reaction was stopped by 2 minutes of incubation in boiling water. The reaction solutions were analyzed by HPLC in the same manner as in Example 4 to assay GnTII activity and the thermal stability of the MBP-hGnTII. The results confirmed that MBP-hGnTII was thermally stable up to 40°C (Figure 4).

### Example 8: Study on Optimal pH for MBP-hGnTII

The MBP-hGnTII obtained in Example 3 was dialyzed against 3 mM MES buffer (pH 6.5, containing 24 mM MnCl₂, 60 mM NaCl, 1.2 mM 2-mercaptoethanol) in order to lower the buffer concentration. 5 µL each of 200 mM buffer solutions with pH levels ranging from 4.0 to 10.5, 1.5 µL of the 10 µM pyridylaminated oligosaccharide represented by Formula 2, and 308 mM UDP-GlcNAc (1 µL) were added to the resulting MBP-hGnTII (25 µL), and reactions were carried out for 5 hours at 37°C. After the reaction, the reaction was stopped by 2 minutes of incubation in boiling water. The reaction solutions were analyzed by HPLC in the same manner as in Example 4 to assay GnTII activity and the optimal pH for the MBP-hGnTII. The results confirmed that MBP-hGnTII was thermally stable up to 40°C (Figure 4). The used buffers were acetate buffer (pH 4.0 to 5.5), cacodylate buffer (pH 5.5 to 7.0), HEPES buffer (pH 7.0 to 8.0), Tricine buffer (pH 8.0 to 8.8), bicine buffer (pH 8.0 to 8.8), and glycine buffer (pH 8.8 to 10.5) .

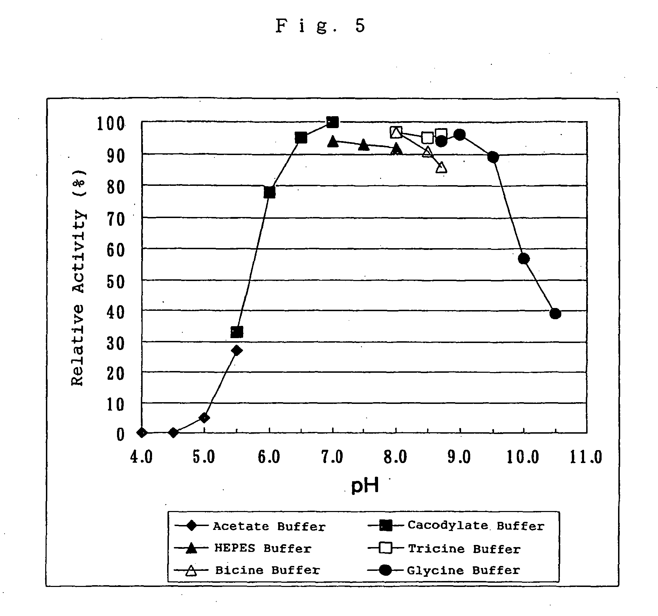

The results confirmed that the optimal pH for MBP-hGnTII is a pH of 6.5 to 9.0 (Figure 5).

### Example 9: Study on pH Stability of MBP-hGnTII

Following the same dialysis as in Example 8, 5 µL each of 200 mM buffer solutions with pH levels of 4.0 to 10.5 were added to the resulting MBP-hGnTII (25 µL), and the resulting solutions were incubated for 7 hours at 25°C. 7.5 µL of 1 M MES buffer (pH 6.5, containing 20 mM MnCl₂, 50 mM NaCl, and 1 mM 2-mercaptoethanol), 2 µL of 10 µM pyridylaminated oligosaccharide represented by Formula 2, and 308 mM UDP-GlcNAc (1 µL) were then added, and a reaction was carried out for 5 hours at 37°C. After the reaction, the reaction was stopped by 2 minutes of incubation in boiling water. The reaction solutions were analyzed by HPLC in the same manner as in Example 4 to assay GnTII activity and the pH stability of the MBP-hGnTII. The used buffers were acetate buffer (pH 4.0 to 5.5), cacodylate buffer (pH 5.5 to 7.0), HEPES buffer (pH 7.0 to 8.0), Tricine buffer (pH 8.0 to 8.8), bicine buffer (pH 8.0 to 8.8), and glycine buffer (pH 8.8 to 10.5).

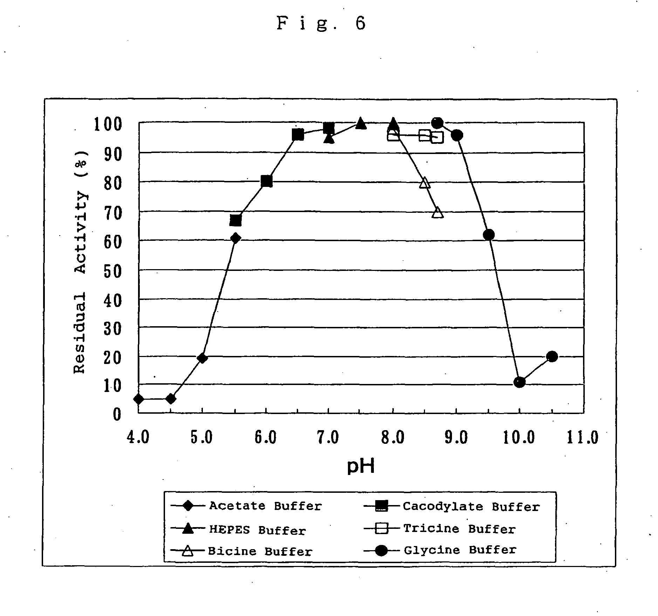

The results confirmed that the MBP-hGnTII was stable at a pH between 5.5 and 9.5 (Figure 6).

### Example 10: Conversion of RNaseB With High Mannose Sugar Chains to RNaseB With Complex-Type Sugar Chains

### (1) Sugar Chain Conversion Reaction

### Step 1: α1,2-Mannosidase Digestion

20 mg of RNaseB was dissolved in 360 µL of 20 mM acetate buffer (pH 5.0), 20 µL of 0.5 U/mL α1,2-mannosidase was added, and a reaction was carried out for 18 hours at 37°C. 15 µL of 0.5 U/mL α1,2-mannosidase was again added thereto, and a reaction was carried out for another 9 hours at 37°C. 5 µL of 0.5 U/mL α1,2-mannosidase was again added thereto, and a reaction was carried out for another 18 hours at 37°C.

300 µL out of the resulting reaction solution was dialyzed against 100 mM MES buffer (pH 6.5, containing 20 mM MnCl₂, 50 mM NaCl, and 1 mM 2-mercaptoethanol). The remaining 100 µL was dialyzed against distilled water for use as a sample to analyze the sugar chain conversion.

### Step 2: β1,2-N-acetylglucosaminyltransferase I Reaction

60 µL of 308 mM UDP-GlcNAc and 660 µL maltose-binding protein-β1,2-N-acetylglucosaminyltransferase I fusion protein (MBP-hGnTI) were added to the α1,2-mannosidase-digested RNaseB (280 µL) obtained in step 1, and a reaction was carried out for 18 hours at 37°C.

666 µL of the resulting reaction solution was dialyzed against 10 mM MES buffer (pH 6.0). The remaining 334 µL was dialyzed against distilled water for use as a sample to analyze the sugar chain conversion.

The MBP-hGnTI obtained by the method in Japanese Unexamined Patent Application (Kokai) 2001-178453 was used.

### Step 3: Jack Bean α-Mannosidase Digestion

94 µL of 100 mM MES buffer (pH 6.0) and 94 µL of Jack bean α-mannose (by Seikagaku Kogyo) were added to the MBP-hGnTI treated RNaseB (750 µL) obtained in step 2, and a reaction was carried out for 15.5 hours at 37°C. After the reaction, another 47 µL of Jack bean α-mannosidase was added, and a reaction was carried out for 13.5 hours at 37°C. After the reaction, another 47 µL of the above α-mannosidase was added, and a reaction was carried out for 10.5 hours at 37°C. After the reaction, another 94 µL of the above α-mannosidase was added, and a reaction was carried out for 7 hours at 37°C. After the reaction, another 94 µL of the above α-mannosidase was added, and a reaction was carried out for 43.5 hours at 37°C, for a total of 90 hours of reaction.

610 µL out of the resulting reaction solution was dialyzed against 100 mM MES buffer (pH 6.5, containing 20 mM MnCl₂, 50 mM NaCl, and 1 mM 2-mercaptoethanol). The remaining 610 µL was dialyzed against distilled water for use as a sample to analyze the sugar chain conversion.

### Step 4: β1, 2-N-acetylglucosaminyltransferase II Reaction

60 µL of 308 mM UDP-GlcNAc and 2.19 mL of the MBP-hGnTII obtained in Example 3 were added to the Jack bean α-mannosidase digested RNaseB (750 µL) obtained in step 3, and a reaction was carried out for 40 hours at 37°C. After the reaction, another 1 mL of MBP-hGnTII was added, and a reaction was carried out for another 11 hours at 37°C. After the reaction, another 1 mL of MBP-hGnTII was added, and a reaction was carried out for another 12.5 hours at 37°C. After the reaction, another 300 µL of MBP-hGnTII was added, and a reaction was carried out for another 11 hours at 37°C. After the reaction, another 500 µL of MBP-hGnTII was added, and a reaction was carried out for another 10.5 hours at 37°C, for a total of 85 hours of reaction.

The resulting reaction solution was dialyzed against distilled water to analyze the sugar chain conversion.

### (2) Analysis of Sugar Chain Conversion

Sugar chains were cut out in the usual manner from samples obtained in steps 1-4 above for fluorescent labeling with 2-aminopyridine. The resulting pyridylaminated oligosaccharides were analyzed by normal phase HPLC.

The conditions for the HPLC are given below.
Column: Amide column Shodex Asahi PAK.
Soln. A: 80% CH₃CN,
Soln. B : 20% CH₃CN,
Gradient : 10 → 50 → 10% Soln. B (5 → 25 → 26 min),
Time: 40 min.
Column temp. : 30 °C

### (a) Analysis of the product in Step 1

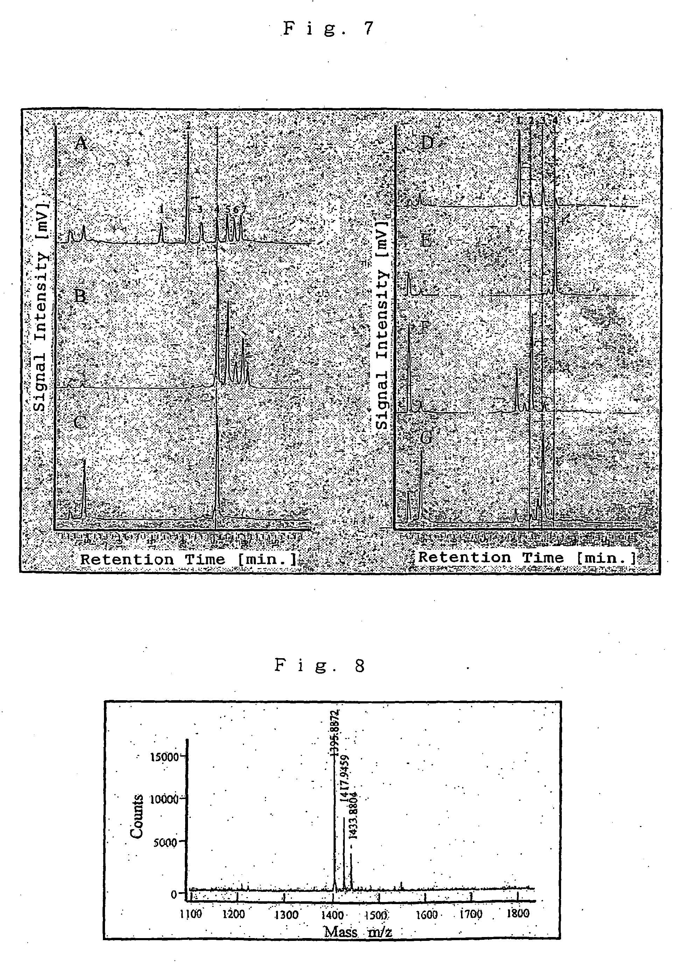

Figure 7A shows the results of analysis for the standard pyridylaminated oligosaccharide. Peaks 1, 2, 3, 4, 5, 6, and 7 correspond to the pyridylaminated oligosaccharides represented by Formulas 4, 5, 9, 10, 11, 12, and 13, respectively.

The results of the sugar chain analysis for untreated RNaseB (Figure 7B) confirmed the presence of various high mannose-type sugar chains corresponding to peaks 4-7 in Figure 7a.

The results of sugar chain analysis of the α1,2-mannosidase digested RNaseB obtained in step 1 (Figure 7C) confirmed that all of the peaks for the various high mannose sugar-type chains detected in Figure 7B had been converted to peak 4 (Formula 10).

### (b) Analysis of the product in Step 2

Figure 7D also shows the results of analysis for the standard pyridylaminated oligosaccharide, where peaks 1, 2, 3, and 4 correspond to the pyridylaminated oligosaccharides of Formulas 5, 6, 3, and 14, respectively.

The results of analysis for the sugar chains of the RNaseB treated with the MBP-hGnTI obtained in step 2 (Figure 7E) revealed only a peak corresponding to peak 4 (Formula 14) in Figure 7D.

### (c) Analysis of the product in Step 3

The results of analysis for the sugar chains of the Jack bean α-mannosidase digested RNaseB obtained in step 3 (Figure 7F) confirmed that the peak corresponding to peak 2 (Formula 6) in Figure 7D was the major reaction product.

### (d) Analysis of the product in Step 4

The results of analysis for the sugar chains of the RNaseB treated with MBP-hGnTII obtained in step 4 (Figure 7G) confirmed that the peak corresponding to peak 3 (Formula 3) in Figure 7D was the primary reaction product.

The results of mass spectrometry analysis of the fractionated main peak in Figure 7G (Figure 8) gave value virtually consistent with the theoretical value for the pyridylaminated oligosaccharide of Formula 3 (1395.32), thus confirming that the sugar chain structure of the RNaseB treated with the β1,2-N-acetylglucosaminyltransferase II obtained in step 4 had the structure represented by Formula 15.

The above results confirmed that RNaseB with various high mannose-type sugar chains could be readily converted enzymatically to RNaseB having complex-type sugar chains.

### Example 11: Preparation of α-Mannosidase II

The livers of 30 mice were homogenized according to the method in J. Biol. Chem., 266:16876 (1991), Golgi body-rich membrane fraction was then obtained. α-mannosidase II was purified from the membrane fraction as follows: solubilization, phase separation in Triton X-114, digestion with α-chymotrypsin, phase separation in Triton X-114, and chromatography on a Mono S (Amersham Pharmacia) column and on a Superose 6 (Amersham Pharmacia) column. The resulting α-mannosidase II fraction was concentrated using Sentricon YM-30, giving 0.5 mL of α-mannosidase II solution.

### Example 12: Conversion of RNaseB With High Mannose-Type Sugar Chains to RNaseB With Complex-Type Sugar Chains

RNaseB sugar chains were converted in the same manner as in Example 10 except that the α-mannosidase II obtained in Example 11 was used instead of the Jack bean α-mannosidase in step 3, and 100 mM MES buffer (pH 6.0) containing 0.1% Triton X-100 was used instead of 100 mM MES buffer (pH 6.0) in step 3. Analysis of the converted RNaseB sugar chains revealed a product corresponding to Formula 3.

### Example 13: Preparation of Immobilized β1, 2-N-Acetylglucosaminyltransferase II

In the same manner as in Example 3, the MBP-hGnTII fusion protein was adsorbed to an amylose resin (New England Biolabs) column (5 mL) and washed with 100 mM MES buffer (pH 6.5, containing 20 mM MnCl₂, 50 mM NaCl, and 1 mM 2-mercaptoethanol, and the resulting resin was used as such for immobilized enzyme.

### Example 14: Preparation of Immobilized β1,2-N-Acetylglucosaminyltransferase I

Recombinant *E. coli* producing the MBP-hGnTI fusion protein as noted in Japanese Unexamined Patent Application (Kokai) 2001-178453 was used to obtain immobilized β1,2-N-acetylglucosaminyltransferase I (5 mL) in the same manner as in Example 11.

### Example 15: Preparation of Immobilized α1,2-Mannosidase

0.5 U of α1,2-mannosidase (Seikagaku Kogyo) and 5 mL of 50 mM HEPES buffer (pH 7.0) containing 20 mg bovine serum albumin were added to 1 mL of NHS activated Sepharose 4FF (Amersham Pharmacia) which had been previously washed with 60 mL of 1 mM hydrochloric acid, and the contents were gently shaken overnight at 4°C to ensure that the enzyme was immobilized. The resin was then filtered off and washed with 5 mL of 50 mM HEPES buffer (pH 7.0). The resin was then placed in 5 mL of 0.1 M Tris-HCl buffer (pH 8.0) and gently shaken for 4 hours at 4°C to block the remaining activated sites in the resin. The resin was then washed with 1 M sodium chloride aqueous solution and distilled water, and 1 mL of the resulting immobilized α1,2-mannosidase was immersed in 50 mM acetate buffer (pH 5.0) and stored at 4°C.

### Example 16: Preparation of Immobilized α-Mannosidase II

1 mL of immobilized α-mannosidase II was obtained in the same manner as in Example 15 except that 0.5 mL of the α-mannosidase II obtained in Example 11 was used instead of the 0.5 U of α1,2-mannosidase (Seikagaku Kogyo).

### Example 17: Preparation of Immobilized β 1,4-Galactosyltransferase

30 U of β1,4-galactosyltransferase (Toyobo), 120 mg of bovine serum albumin, and 10 mL of 50 mM HEPES buffer (pH 7.0) containing 1 mM of uridine-5'-diphosphogalactose (UDP-Gal), 5 mM N-acetylglucosamine, and 25 mM manganese chloride were added to 3 mL CNBr-activated Sepharose 4B (Amersham Pharmacia) which had been previously washed with 100 mL of 1 mM hydrochloric acid, and the contents were gently shaken overnight at 4°C to ensure that the enzyme was immobilized. The resin was then filtered off and washed with 10 mL of 50 mM HEPES buffer (pH 7.0). The resin was then placed in 10 mL of 0.1 M Tris-HCl buffer (pH 8.0) and gently shaken for 4 hours at 4°C to block the remaining activated sites in the resin. The resin was then washed with 1 M sodium chloride aqueous solution and distilled water, and 3 mL of the resulting immobilized β1,4-galactosyltransferase was immersed in 25 mM HEPES buffer (pH 7.0) containing UDP-Gal (1 mM) and 5 mM manganese chloride, and stored at 4°C.

### Example 18: Preparation of Immobilized α 2,6-Sialyltransferase

1.2 U of α2,6-sialyltransferase (Toyobo), 75 mg bovine serum albumin, and 10 mL of 50 mM HEPES buffer (pH 7.0) containing 1 mM cytidine-5'-diphosphate and 5 mM N-acetyllactosamine were added to 1.0 g of NHS activated Sepharose 4FF (Amersham Pharmacia) which had been previously washed with 100 mL of 1 mM hydrochloric acid, and the contents were gently shaken overnight at 4°C to ensure that the enzyme was immobilized. The resin was then filtered off and washed with 10 mL of 50 mM HEPES buffer (pH 7.0). The resin was then placed in 10 mL of 0.1 M Tris-HCl buffer (pH 8.0) and gently shaken for 4 hours at 4°C to block the remaining activated sites in the resin. The resin was then washed with 1 M sodium chloride aqueous solution and distilled water, and 3 mL of the resulting immobilized α2,6-sialyltransferase was immersed in 25 mM HEPES buffer (pH 7.0) containing 1 mM cytidine-5'-monophospho-N-acetylneuraminic acid (CMP-NeuAc), and stored at 4°C.

### Example 19: Preparation of Immobilized α-Mannosidase

Immobilized α-mannosidase was obtained in the same manner as in Example 11 using 30 U α-mannosidase (from Jack beans, by Seikagaku Kogyo), and was immersed in 50 mM HEPES buffer (pH 7.0) containing 0.01 mM zinc acetate, and stored at 4°C.

### Example 20: Use of Immobilized Enzyme for Conversion of RNaseB High Mannose-Type Sugar Chains to Hybrid-Type Sugar Chains

### (1) Sugar Chain Conversion Reaction

### Step 1: Trimming High Mannose-Type Sugar Chains With Immobilized α1,2-Mannosidase

100 mg RNaseB (Sigma) was dissolved in 2 mL of 20 mM acetate buffer (pH 5.0), 1 mL of the immobilized α1,2-mannosidase obtained in Example 15 was added thereto, and a reaction was carried out as the contents were gently shaken for 24 hours at 37°C. After the reaction, the immobilized 1,2-mannosidase was filtered off, and the immobilized α1,2-mannosidase was washed with 5 mL of distilled water. The reaction solution and the wash were combined, the mixture was introduced into a dialysis membrane (by Spectrum) with a 3.5 kD molecular weight cut off and was dialyzed against distilled water, and it was then lyophilized, giving RNaseB (87 mg) in which the target sugar chains had been trimmed.

### Step 2: Elongation of Sugar Chains with Immobilized β1,2-N-acetylglucosaminyltransferase I

The RNaseB with trimmed sugar chains (15 mg) was dissolved in 1 mL of 100 mM MES buffer (pH 6.5) containing 10 mM uridine-5'-diphospho-N-acetylglucosamine (UDP-GlcNAc), 20 mM manganese chloride, 10 mM sodium chloride, and 0.2 mM 2-mercaptoethanol, 1 mL of the immobilized β1,2-N-acetylglucosaminyltransferase I obtained in Example 14 was added, and a reaction was carried out for 24 hours as the contents were gently shaken at 25°C. After the reaction, the immobilized β1, 2-N-acetylglucosaminyltransferase I was filtered off, and the immobilized β1,2-N-acetylglucosaminyl transferase I was washed with 5 mL of distilled water. The reaction solution and the wash were combined, and the mixture was introduced into a dialysis membrane (by Spectrum) with a 3.5 kD molecular weight cut off and was dialyzed against 50 mM HEPES buffer (pH 7.5) containing 10 mM manganese chloride. After the dialysis, it was concentrated to 1 mL using Ultrafree-MC (Millipore, molecular weight cut off 5,000).

### Step 3: Elongation of Sugar Chains with Immobilized β1,4-Galactosyltransferase

UDP-Gal was added to a concentration of 10 mM in the solution obtained in step 2, 1 mL of the immobilized β1,4-galactosyltransferase obtained in Example 17 was then added, and a reaction was carried out for 24 hours as the contents were gently shaken at 25°C. After the reaction, a 1 mL solution of the target material was obtained in the same manner as in step 2.

### Step 4: Elongation of Sugar Chains with Immobilized α2,6-Sialyltransferase

CMP-NeuAc was added to a concentration of 10 mM in 1 mL of the solution obtained in step 3, 1 mL of the immobilized α2,6-sialyltransferase obtained in Example 18 was then added, and a reaction was carried out for 48 hours as the contents were gently shaken at 25°C. The reaction was followed by dialysis against distilled water in the same manner as in step 2, and then by lyophilization, giving 10 mg of the target material.

### (2) Analysis of Sugar Chain Conversion

Sugar chains were cut out in the usual manner from samples obtained in steps 1-4 above for fluorescent labeling with 2-aminopyridine. The resulting pyridylaminated oligosaccharides were analyzed by normal phase HPLC, confirming that the major component of the sugar chain structure in the lyophilized product obtained in step 4 was the structure represented by Formula 16.

### Example 21: Use of Immobilized Enzyme for Conversion of RNaseB High Mannose-Type Sugar Chains to Complex-Type Sugar Chains

### (1) Sugar Chain Conversion Reaction

### Step 1: Trimming High Mannose-Type Sugar Chains With Immobilized α1,2-Mannosidase

RNaseB with trimmed sugar chains (87 mg) was obtained in the same manner as in step 1 in Example 20.

### Step 2: Elongation of Sugar Chains with Immobilized β1,2-N-acetylglucosaminyltransferase I

The RNaseB with trimmed sugar chains (15 mg) was dissolved in 1 mL of 100 mM MES buffer (pH 6.5) containing UDP-GlcNAc (10 mM), 20 mM manganese chloride, 10 mM sodium chloride, and 0.2 mM 2-mercaptoethanol, the immobilized β1,2-N-acetylglucosaminyltransferase I (1 mL) obtained in Example 14 was added, and a reaction was carried out for 24 hours as the contents were gently shaken at 25°C. After the reaction, the immobilized β1,2-N-acetylglucosaminyltransferase I was filtered off, and the immobilized β1,2-N-acetylglucosaminyltransferase I was washed with 5 mL of distilled water. The reaction solution and the wash were combined, and the mixture was introduced into a dialysis membrane (by Spectrum) with a 3.5 kD molecular weight cut off and was dialyzed against 100 mM MES buffer (pH 6.0) containing 0.01 mM zinc acetate. After the dialysis, it was concentrated to 1 mL using Ultrafree-MC (Millipore, molecular weight cut off 5,000).

### Step 3: Trimming Sugar Chains With Immobilized α-Mannosidase

1 mL of the immobilized α-mannosidase obtained in Example 19 was added to 1 mL of the reaction solution obtained in step 2, and a reaction was carried out for 24 hours as the contents were gently shaken at 25°C. After the reaction, a 1 mL solution of the target material was obtained in the same manner as in step 2 except dialysis against 100 mM MES buffer (pH 6.5) containing 20 mM manganese chloride, 10 mM sodium chloride, and 0.2 mM 2-mercaptoethanol.

### Step 4: Elongation of Sugar Chains with Immobilized β1,2-N-acetylglucosaminyltransferase II

UDP-GlcNAc was added to a concentration of 10 mM in the solution obtained in step 3, 1 mL of the immobilized β1,2-N-acetylglucosaminyltransferase II obtained in Example 13 was added, and a reaction was carried out for 48 hours as the contents were gently shaken at 25°C. After the reaction, a 1 mL solution of the target material was obtained in the same manner as in step 2 except dialysis against 50 mM HEPES buffer (pH 7.5) containing 10 mM manganese chloride.

### Step 5: Elongation of Sugar Chains with Immobilized β1,4-Galactosyltransferase

The sugar chains were elongated in the same manner as in step 3 in Example 20, giving a 1 mL solution of the target material.

### Step 6: Elongation of Sugar Chains with Immobilized α2,6-Sialyltransferase

The sugar chains were elongated in the same manner as in step 4 in Example 20 except that the reaction time was 72 hours, giving 6 mg of the target material.

### (2) Analysis of Sugar Chain Conversion

Sugar chains were cut out in the usual manner from samples obtained in steps 1-6 above for fluorescent labeling with 2-aminopyridine. The resulting pyridylaminated oligosaccharides were analyzed by normal phase HPLC, confirming that the major component of the sugar chain structure in the lyophilized product obtained in step 6 was the structure represented by Formula 17.

### Example 22: Use of Immobilized Enzyme for Conversion of High Mannose-Type Sugar Chains to Complex-Type Sugar Chains

RNaseB sugar chains were converted in the same manner as in Example 21 except that the immobilized α-mannosidase II obtained in Example 16 was used instead of the immobilized α-mannosidase obtained in step 3 of Example 21. Analysis of the sugar chain structure of converted sugar chains which had been cut out confirmed that the major component was the structure represented by Formula 17.

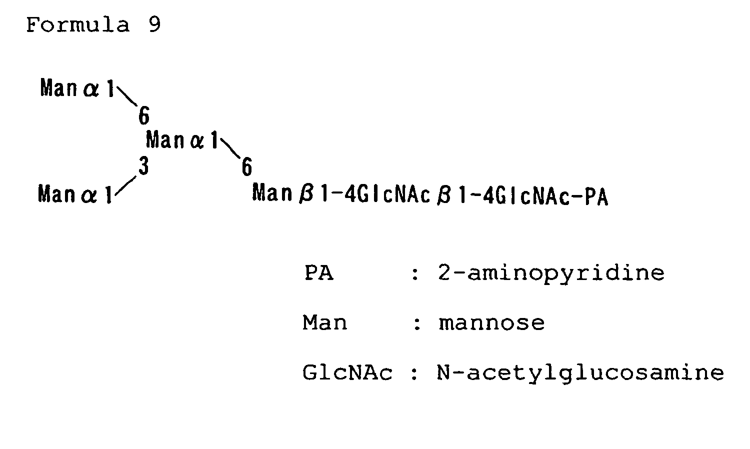

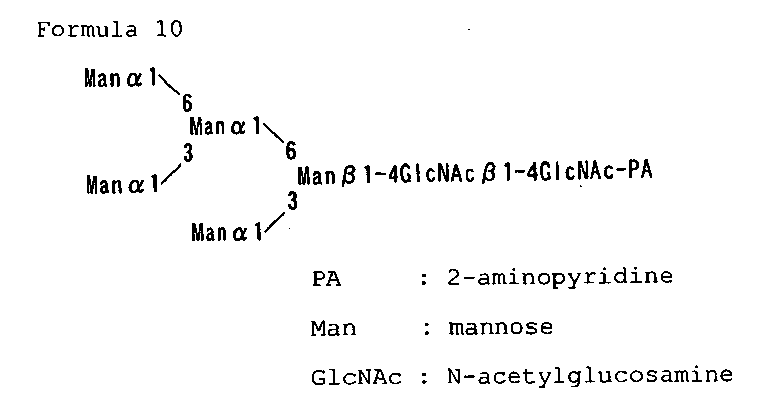

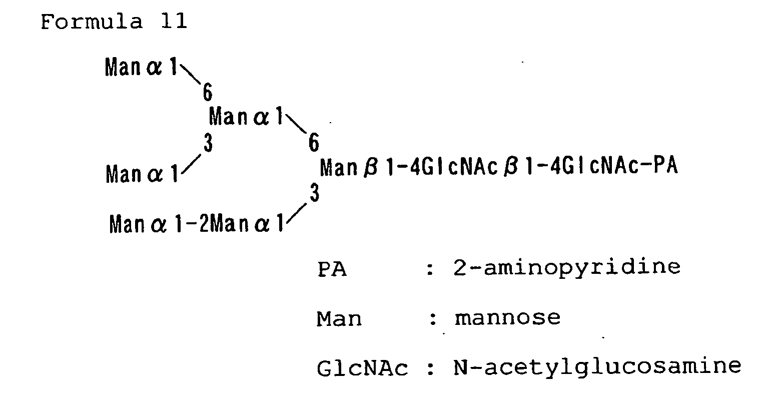

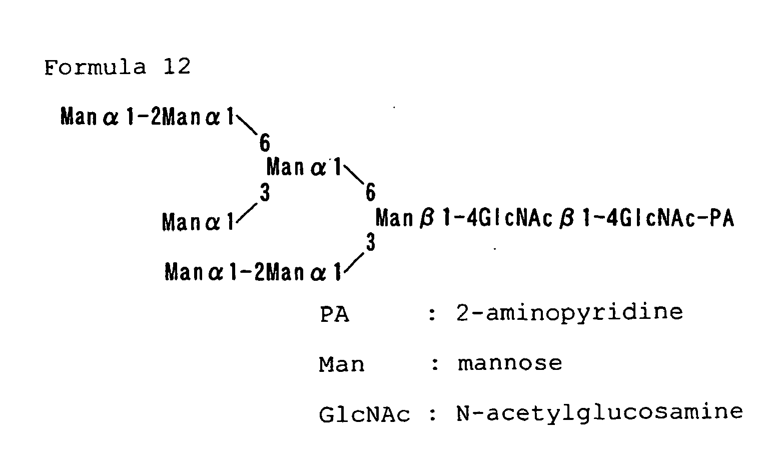

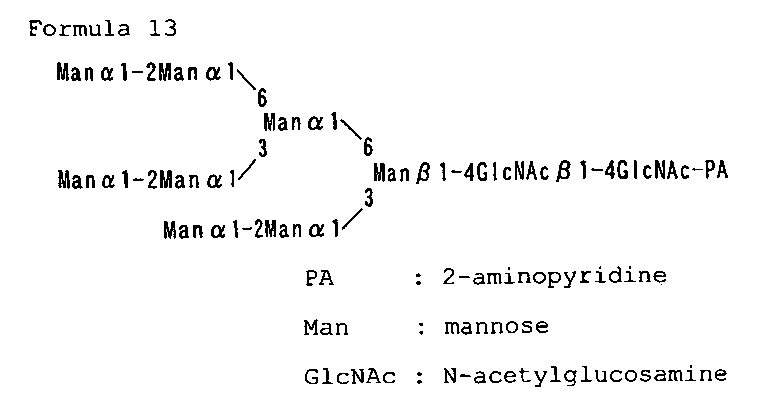

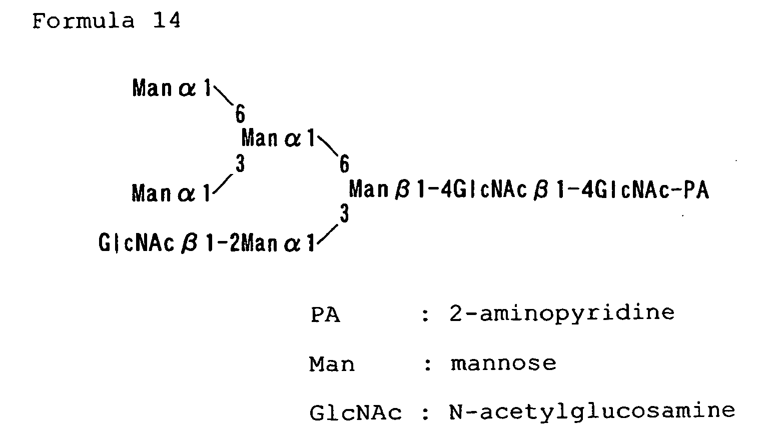

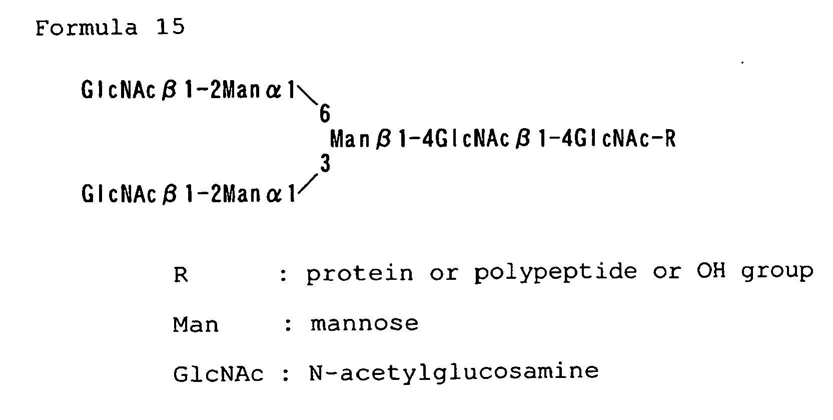

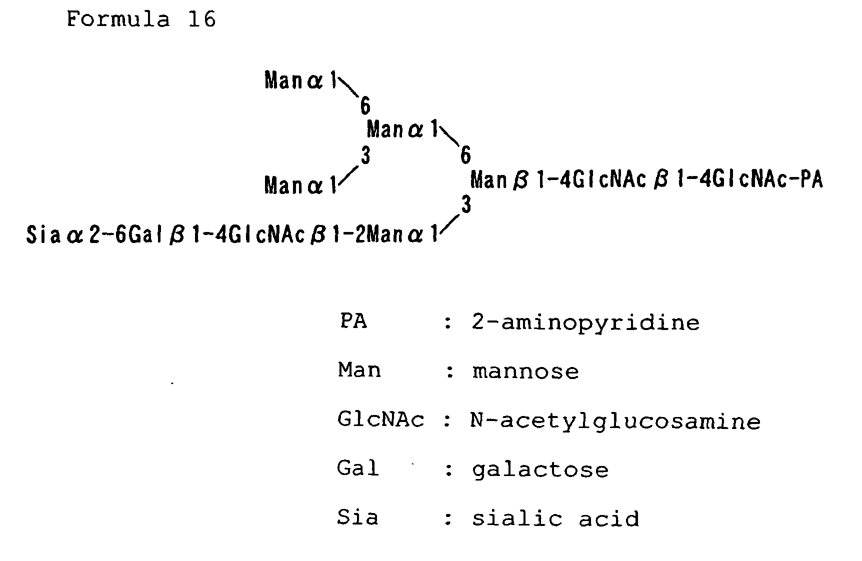

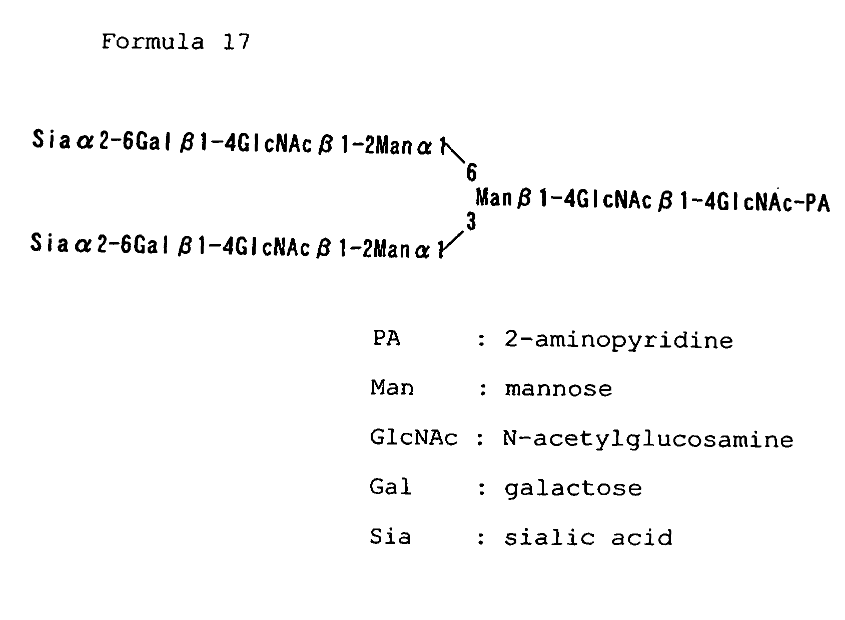

In the method of the present invention, a fusion protein having N-acetylglucosaminyltransferase II activity can be readily produced efficiently in large amounts in the form of a soluble protein. In addition, the N-acetylglucosaminyltransferase II itself can be readily obtained from the fusion protein, and the resulting N-acetylglucosaminyltransferase II allow to synthesize useful sugar cains and obtain anti N-acetylglucosaminyltransferase II antibody which can be useful for diagnostics.

## Claims

1. A recombinant fusion protein of a maltose-binding protein and β1,2-N-acetylglucosaminyltransferase II.

2. The fusion protein of Claim 1, wherein the β1,2-N-acetylglucosaminyltransferase II is derived from humans.

3. The fusion protein of Claim 1, wherein the β1,2-N-acetylglucosaminyltransferase II is (a) a protein comprising the amino acid sequence in SEQ ID NO. 2, or (b) a protein with β1,2-N-acetylglucosaminyl transferase II activity, comprising the amino acid sequence in (a) above with one or more amino acids deleted, substituted, or added.

4. The fusion protein of Claim 3, wherein the β1,2-N-acetylglucosaminyltransferase II comprises at least the amino acid sequence 20-447 in the amino acid sequence in SEQ ID NO. 2.

5. The fusion protein of Claim 1, wherein the β1,2-N-acetylglucosaminyltransferase II comprises an amino acid sequence in which amino acids corresponding to part or all of the protein transmembrane domain have been deleted.

6. The fusion protein of Claim 1, comprising a protease recognition site between the sugar-binding protein and the β1,2-N-acetylglucosaminyltransferase II.

7. DNA coding for a fusion protein according to any one of Claims 1 through 6.

8. An expression vector comprising DNA according to Claim 7.

9. A non-human transformant resulting from transformation with an expression vector according to Claim 8.

10. A method for producing a maltose-binding protein/β1,2-N-acetylglucosaminyltransferase II fusion protein, comprising the following steps of:
(1) transforming *E. coli* using an expression vector to which DNA coding for a maltose-binding protein and DNA coding for β1,2-N-acetylglucosaminyltransferase II have been ligated in such a way that the two proteins are expressed in the form of a fusion protein under the control of a promoter capable of functioning in *E. coli*;
(2) cultivating the resulting transformants to produce a fusion protein of the maltose-binding protein and the β1,2-N-acetylglucosaminyltransferase II; and
(3) isolating the fusion protein from the resulting culture.

11. The method of Claim 10, wherein the β1,2-N-acetylglucosaminyltransferase II is derived from humans.

12. The method of Claim 11, wherein the DNA coding for the β1,2-N-acetylglucosaminyltransferase II comprises a nucleotide sequence coding for at least the amino acid sequence 29-447 in the amino acid sequence in SEQ ID NO:2.

13. The method of Claim 11, wherein the DNA coding for the β1,2-N-acetylglucosaminyltransferase II comprises at least the nucleotide sequence 85-1341 in the nucleotide sequence in SEQ ID NO:1.

14. The method of Claim 10, wherein the DNA coding for the β1,2-N-acetylglucosaminyltransferase II comprises a nucleotide sequence coding for an amino acid sequence in which amino acids corresponding to part or all of the protein transmembrane domain have been deleted.

15. The method of Claim 10, wherein the DNA coding for the β1,2-N-acetylglucosaminyltransferase II comprises the nucleotide sequence in SEQ ID NO. 1 from which amino acids corresponding to part or all of the protein transmembrane domain have been deleted.

16. The method of Claim 10, wherein the DNA coding for the maltose-binding protein is derived from pMAL-p2 or pMAL-c2.

17. The method of Claim 10, wherein the fusion protein is isolated in the presence of divalent metal ions from the culture obtained in step (3).

18. The method of Claim 17, wherein the divalent metal is manganese.

19. A method for producing β1,2-N-acetyl glucosaminyltransferase II, comprising the step of isolating the β1,2-N-acetylglucosaminyltransferase II after eliminating the maltose-binding protein portion from the fusion protein obtained by a method according to any of Claims 10 through 18.

20. The method of Claim 19, **characterized in that** the DNA coding for the sugar-binding protein comprises a nucleotide sequence coding for a protease recognition site on the C terminal end of the protein, and the sugar-binding protein portion is eliminated from the fusion protein through the action of a protease.

21. The method of Claim 20, wherein the protease is blood coagulation factor Xa.

22. A method for converting sugar chains on glycoproteins to complex type sugar chains, comprising steps (1) through (3) below:
(1) allowing a glycosidase to act on glycoprotein sugar chains;
(2) allowing β1,2-N-acetylglucosaminyltransferase I to act, in the presence of UDP-GIcNAc, on the glycoproteins obtained in step (1); and
(3) allowing β1,2-N-acetylglucosaminyltransferase II, being a recombinant fusion protein according to any one of claims 1 through 6, to act, in the presence of UDP-GIcNAc, on the glycoproteins obtained in step (2).

23. The method of Claim 22, **characterized in that** at least one kind of glycosyltransferase is furthermore allowed to act after step (3).

24. The method of Claim 23, wherein the glycosyltransferase is at least one selected from sialyltransferase, fucosyltransferase, galactosyltransferase, and N-acetylglucosaminyltransferase.

25. The method of Claim 23, wherein at least one glycosyltransferase is an immobilized enzyme.

26. The method of Claim 22, wherein the glycosidase is at least one selected from galactosidase, N-acetylglucosaminidase, fucosidase, sialidase, xylosidase, and mannosidase.

27. The method of Claim 22, wherein at least one of the glycosidase, β1,2-N-acetylglucosaminyltransferase I, or β1,2-N-acetylglucosaminyltransferase II is an immobilized enzyme.

28. The method of Claim 22, wherein the glycoprotein is naturally derived.

29. The method of Claim 22, wherein the glycoprotein is recombinant.

30. The method of claim 22 comprising steps (1) through (4) below:
(1) allowing a glycosidase to act on glycoprotein sugar chains;
(2) allowing β1,2-N-acetylglucosaminyltransferase I to act, in the presence of UDP-GIcNAc, on the glycoproteins obtained in step (1);
(3) allowing α-mannosidase to act on the glycoproteins obtained in step (2); and
(4) allowing β1,2-N-acetylglucosaminyltransferase II, being a recombinant fusion protein according to any one of claims 1 through 6, to act, in the presence of UDP-GIcNAc, on the glycoproteins obtained in step (3).

31. The method of Claim 30, **characterized in that** at least one kind of glycosyltransferase is furthermore allowed to act after step (4).

32. The method of Claim 31, wherein the glycosyltransferase is at least one selected from sialyltransferase, fucosyltransferase, galactosyltransferase, and N-acetylglucosaminyltransferase.

33. The method of Claim 31, wherein at least one glycosyltransferase is an immobilized enzyme.

34. The method of Claim 30, wherein the glycosidase is at least one selected from galactosidase, N-acetylglucosaminidase, fucosidase, sialidase, xylosidase, and mannosidase.

35. The method of Claim 30, wherein the glycosidase is α-mannosidase.

36. The method of Claim 30, wherein the glycosidase is α1,2-mannosidase.

37. The method of Claim 30, wherein the α-mannosidase is α-mannosidase II.

38. The method of Claim 30, wherein at least one of the glycosidase, β1,2-N-acetyl glucosaminyltransferase I, α-mannosidase, or β1,2-N-acetylglucosaminyltransferase II is an immobilized enzyme.

39. The method of Claim 30, wherein the glycoprotein is naturally derived.

40. The method of Claim 30, wherein the glycoprotein is recombinant.

41. A method for converting sugar chains on glycoproteins to complex type sugar chains, comprising the following steps (1) through (3):
(1) allowing β1,2-N-acetylglucosaminyi transferase I to act, in the presence of UDP-GIcNAc, on glycoproteins having a structure wherein part or all of the sugar chain structures on the glycoproteins serve as the substrate for the β1,2-N-acetylglucosaminyltransferase I;
(2) allowing α-mannosidase to act on the glycoproteins obtained in step (1); and
(3) allowing β1,2-N-acetylglucosaminyltransferase II being a recombinant fusion protein according to any one of claims 1 through 6 to act, in the presence of UDP-GIcNAc, on the glycoproteins obtained in step (2).

42. The method of Claim 41 for converting sugar chains on glycoproteins to complex type sugar chains, **characterized in that** at least one kind of glycosyltransferase is furthermore allowed to act after step (3).

43. The method of Claim 42, wherein the glycosyltransferase is at least one selected from sialyltransferase, fucosyltransferase, galactosyltransferase, and N-acetylglucosaminyltransferase.

44. The method of Claim 42, wherein at least one glycosyltransferase is an immobilized enzyme.

45. The method of Claim 41, wherein the α-mannosidase is α-mannosidase II.

46. The method of Claim 41, wherein at least one of the β1,2-N-acetylglucosaminyltransferase I, α-mannosidase, or β1,2-N-acetylglucosaminyltransferase II is an immobilized enzyme.

47. The method of Claim 41, wherein the glycoprotein is naturally derived.

48. The method of Claim 41, wherein the glycoprotein is recombinant.

49. A method for converting hybrid-type sugar chains on glycoproteins to complex-type sugar chains, comprising steps (1) and (2) below:
(1) allowing a glycosidase to act on hybrid-type sugar chains of glycoproteins; and
(2) allowing β1,2-N-acetylglucosaminyltransferase II, being a recombinant fusion protein according to any one of claims 1 through 6, to act, in the presence of UDP-GIcNAc, on the glycoproteins obtained in step (1).

50. The method of Claim 49, **characterized in that** at least one kind of glycosyltransferase is furthermore allowed to act after step (2).

51. The method according to Claim 50, wherein the glycosyltransferase is at least one selected from sialyltransferase, fucosyltransferase, galactosyltransferase, and N-acetylglucosaminyltransferase.

52. The method of Claim 50, wherein at least one glycosyltransferase is an immobilized enzyme.

53. The method of Claim 49, wherein the glycosidase is at least one selected from mannosidase, xylosidase, fucosidase, and β1,4-N-acetylglucosaminidase.

54. The method of Claim 49, wherein at least one of the glycosidase or β1,2-N-acetylglucosaminyltransferase II is an immobilized enzyme.

55. The method of Claim 49, wherein the glycoprotein is naturally derived.

56. The method of Claim 49, wherein the glycoprotein is recombinant.

## Patentansprüche

1. Rekombinantes Fusionsprotein aus einem maltosebindenden Protein und β-1,2-N-Acetylglucosaminyltransferase II.

2. Fusionsprotein gemäß Anspruch 1, wobei die β-1,2-N-Acetylglucosaminyltransferase II vom Menschen abgeleitet ist.

3. Fusionsprotein gemäß Anspruch 1, wobei die β-1,2-N-Acetylglucosaminyltransferase II Folgendes ist: (a) ein Protein, das die Aminosäuresequenz in SEQ ID Nr. 2 umfasst, oder (b) ein Protein mit β-1,2-N-Acetylglucosaminyltransferase-II-Aktivität, das die Aminosäuresequenz in (a) oben umfasst, bei der eine oder mehrere Aminosäuren deletiert, substituiert oder addiert sind.

4. Fusionsprotein gemäß Anspruch 3, wobei die β-1,2-N-Acetylglucosaminyltransferase II wenigstens die Aminosäuresequenz 20-447 in der Aminosäuresequenz in SEQ ID Nr. 2 umfasst.

5. Fusionsprotein gemäß Anspruch 1, wobei die β-1,2-N-Acetylglucosaminyltransferase II eine Aminosäuresequenz umfasst, bei der Aminosäuren, die einem Teil oder der gesamten Protein-Transmembrandomäne entsprechen, deletiert sind.

6. Fusionsprotein gemäß Anspruch 1, das eine Protease-Erkennungsstelle zwischen dem zuckerbindenden Protein und der β-1,2-N-Acetylglucosaminyltransferase II umfasst.

7. DNA, die für ein Fusionsprotein gemäß einem der Ansprüche 1 bis 6 codiert.

8. Expressionsvektor, der DNA gemäß Anspruch 7 umfasst.

9. Nichthumane Transformante, die sich aus einer Transformation mit einem Expressionsvektor gemäß Anspruch 8 ergibt.

10. Verfahren zur Herstellung eines Fusionsproteins aus einem maltosebindenden Protein und β-1,2-N-Acetylglucosaminyltransferase II, das die folgenden Schritte umfasst:
(1) Transformieren von *E. coli* unter Verwendung eines Expressionsvektors, an den DNA, die für ein maltosebindendes Protein codiert, und DNA, die für β-1,2-N-Acetylglucosaminyltransferase II codiert, so ligiert wurden, dass die beiden Proteine in Form eines Fusionsproteins unter Kontrolle eines Promotors, der in *E. coli* funktionieren kann, exprimiert werden;
(2) Kultivieren der resultierenden Transformanten, so dass ein Fusionsprotein aus einem maltosebindenden Protein und β-1,2-N-Acetylglucosaminyltransferase II produziert wird; und
(3) Isolieren des Fusionsproteins aus der resultierenden Kultur.

11. Verfahren gemäß Anspruch 10, wobei die β-1,2-N-Acetylglucosaminyltransferase II vom Menschen abgeleitet ist.

12. Verfahren gemäß Anspruch 11, wobei die für die β-1,2-N-Acetylglucosaminyltransferase II codierende DNA eine Nucleotidsequenz umfasst, die für wenigstens die Aminosäuresequenz 29-447 in der Aminosäuresequenz in SEQ ID Nr. 2 codiert.

13. Verfahren gemäß Anspruch 11, wobei die für die β-1,2-N-Acetylglucosaminyltransferase II codierende DNA wenigstens die Nucleotidsequenz 85-1341 in der Nucleotidsequenz in SEQ ID Nr. 1 umfasst.

14. Verfahren gemäß Anspruch 10, wobei die für die β-1,2-N-Acetylglucosaminyltransferase II codierende DNA eine Nucleotidsequenz umfasst, die für eine Aminosäuresequenz codiert, bei der Aminosäuren, die einem Teil oder der gesamten Protein-Transmembrandomäne entsprechen, deletiert sind.

15. Verfahren gemäß Anspruch 10, wobei die für die β-1,2-N-Acetylglucosaminyltransferase II codierende DNA die Nucleotidsequenz in SEQ ID Nr. 1 umfasst, aus der Aminosäuren, die einem Teil oder der gesamten Protein-Transmembrandomäne entsprechen, deletiert sind.

16. Verfahren gemäß Anspruch 10, wobei die für das maltosebindende Protein codierende DNA von pMAL-p2 oder pMAL-c2 abgeleitet ist.

17. Verfahren gemäß Anspruch 10, wobei das Fusionsprotein in Gegenwart von zweiwertigen Metallionen aus der in Schritt (3) erhaltenen Kultur isoliert wird.

18. Verfahren gemäß Anspruch 17, wobei es sich bei dem zweiwertigen Metall um Mangan handelt.

19. Verfahren zur Herstellung von β-1,2-N-Acetylglucosaminyltransferase II, das den Schritt des Isolierens der β-1,2-N-Acetylglucosaminyltransferase II umfasst, nachdem aus dem nach einem Verfahren gemäß einem der Ansprüche 10 bis 18 erhaltenen Fusionsprotein der Teil des maltosebindenden Proteins entfernt wurde.

20. Verfahren gemäß Anspruch 19, **dadurch gekennzeichnet, dass** die für das zuckerbindende Protein codierende DNA eine Nucleotidsequenz umfasst, die für eine Protease-Erkennungsstelle am C-terminalen Ende des Proteins codiert, und der zuckerbindende Proteinteil durch die Wirkung einer Protease aus dem Fusionsprotein entfernt wird.

21. Verfahren gemäß Anspruch 20, wobei es sich bei der Protease um Blutgerinnungsfaktor Xa handelt.

22. Verfahren zur Umwandlung von Zuckerketten an Glycoproteinen in Zuckerketten des Komplextyps, das die folgenden Schritte (1) bis (3) umfasst:
(1) Einwirkenlassen einer Glycosidase auf Glycoprotein-Zuckerketten;
(2) Einwirkenlassen von β-1,2-N-Acetylglucosaminyltransferase I auf die in Schritt (1) erhaltenen Glycoproteine in Gegenwart von UDP-GIcNAc; und
(3) Einwirkenlassen von β-1,2-N-Acetylglucosaminyltransferase II, bei der es sich um ein rekombinantes Fusionsprotein gemäß einem der Ansprüche 1 bis 6 handelt, auf die in Schritt (2) erhaltenen Glycoproteine in Gegenwart von UDP-GIcNAc.

23. Verfahren gemäß Anspruch 22, **dadurch gekennzeichnet, dass** nach Schritt (3) weiterhin noch wenigstens eine Art von Glycosyltransferase einwirken gelassen wird.

24. Verfahren gemäß Anspruch 23, wobei die Glycosyltransferase wenigstens eine ist, die aus Sialyltransferase, Fucosyltransferase, Galactosyltransferase und N-Acetylglucosaminyltransferase ausgewählt ist.

25. Verfahren gemäß Anspruch 23, wobei wenigstens eine Glycosyltransferase ein immobilisiertes Enzym ist.

26. Verfahren gemäß Anspruch 22, wobei die Glycosidase wenigstens eine ist, die aus Galactosidase, N-Acetylglucosaminidase, Fucosidase, Sialidase, Xylosidase und Mannosidase ausgewählt ist.

27. Verfahren gemäß Anspruch 22, wobei wenigstens eines der Enzyme Glycosidase, β-1,2-N-Acetylglucosaminyltransferase I oder β-1,2-N-Acetylglucosaminyltransferase II ein immobilisiertes Enzym ist.

28. Verfahren gemäß Anspruch 22, wobei das Glycoprotein natürlichen Ursprungs ist.

29. Verfahren gemäß Anspruch 22, wobei das Glycoprotein rekombinant ist.

30. Verfahren gemäß Anspruch 22, das die folgenden Schritte (1) bis (4) umfasst:
(1) Einwirkenlassen einer Glycosidase auf Glycoprotein-Zuckerketten;
(2) Einwirkenlassen von β-1,2-N-Acetylglucosaminyltransferase I auf die in Schritt (1) erhaltenen Glycoproteine in Gegenwart von UDP-GIcNAc;
(3) Einwirkenlassen von α-Mannosidase auf die in Schritt (2) erhaltenen Glycoproteine; und
(4) Einwirkenlassen von β-1,2-N-Acetylglucosaminyltransferase II, bei der es sich um ein rekombinantes Fusionsprotein gemäß einem der Ansprüche 1 bis 6 handelt, auf die in Schritt (3) erhaltenen Glycoproteine in Gegenwart von UDP-GIcNAc.

31. Verfahren gemäß Anspruch 30, **dadurch gekennzeichnet, dass** nach Schritt (4) weiterhin noch wenigstens eine Art von Glycosyltransferase einwirken gelassen wird.

32. Verfahren gemäß Anspruch 31, wobei die Glycosyltransferase wenigstens eine ist, die aus Sialyltransferase, Fucosyltransferase, Galactosyltransferase und N-Acetylglucosaminyltransferase ausgewählt ist.

33. Verfahren gemäß Anspruch 31, wobei wenigstens eine Glycosyltransferase ein immobilisiertes Enzym ist.

34. Verfahren gemäß Anspruch 30, wobei die Glycosidase wenigstens eine solche ist, die aus Galactosidase, N-Acetylglucosaminidase, Fucosidase, Sialidase, Xylosidase und Mannosidase ausgewählt ist.

35. Verfahren gemäß Anspruch 30, wobei es sich bei der Glycosidase um α-Mannosidase handelt.

36. Verfahren gemäß Anspruch 30, wobei es sich bei der Glycosidase um α-1,2-Mannosidase handelt.

37. Verfahren gemäß Anspruch 30, wobei es sich bei der α-Mannosidase um α-Mannosidase II handelt.

38. Verfahren gemäß Anspruch 30, wobei wenigstens eines der Enzyme Glycosidase, β-1,2-N-Acetylglucosaminyltransferase I, α-Mannosidase oder β-1,2-N-Acetylglucosaminyltransferase II ein immobilisiertes Enzym ist.

39. Verfahren gemäß Anspruch 30, wobei das Glycoprotein natürlichen Ursprungs ist.

40. Verfahren gemäß Anspruch 30, wobei das Glycoprotein rekombinant ist.

41. Verfahren zur Umwandlung von Zuckerketten an Glycoproteinen in Zuckerketten des Komplextyps, das die folgenden Schritte (1) bis (3) umfasst:
(1) Einwirkenlassen von β-1,2-N-Acetylglucosaminyltransferase I auf Glycoproteine mit einer Struktur, bei der ein Teil oder alle Zuckerkettenstrukturen an den Glycoproteinen als Substrat für die β-1,2-N-Acetylglucosaminyltransferase I dienen, in Gegenwart von UDP-GIcNAc;
(2) Einwirkenlassen von α-Mannosidase auf die in Schritt (1) erhaltenen Glycoproteine; und
(3) Einwirkenlassen von β-1,2-N-Acetylglucosaminyltransferase II, bei der es sich um ein rekombinantes Fusionsprotein gemäß einem der Ansprüche 1 bis 6 handelt, auf die in Schritt (2) erhaltenen Glycoproteine in Gegenwart von UDP-GIcNAc.

42. Verfahren gemäß Anspruch 41 zur Umwandlung von Zuckerketten an Glycoproteinen in Zuckerketten des Komplextyps, **dadurch gekennzeichnet, dass** nach Schritt (3) weiterhin noch wenigstens eine Art von Glycosyltransferase einwirken gelassen wird.

43. Verfahren gemäß Anspruch 42, wobei die Glycosyltransferase wenigstens eine ist, die aus Sialyltransferase, Fucosyltransferase, Galactosyltransferase und N-Acetylglucosaminyltransferase ausgewählt ist.

44. Verfahren gemäß Anspruch 42, wobei wenigstens eine Glycosyltransferase ein immobilisiertes Enzym ist.

45. Verfahren gemäß Anspruch 41, wobei es sich bei der α-Mannosidase um α-Mannosidase II handelt.

46. Verfahren gemäß Anspruch 41, wobei wenigstens eines der Enzyme β-1,2-N-Acetylglucosaminyltransferase I, α-Mannosidase oder β-1,2-N-Acetylglucosaminyltransferase II ein immobilisiertes Enzym ist.

47. Verfahren gemäß Anspruch 41, wobei das Glycoprotein natürlichen Ursprungs ist.

48. Verfahren gemäß Anspruch 41, wobei das Glycoprotein rekombinant ist.

49. Verfahren zur Umwandlung von Zuckerketten des Hybridtyps an Glycoproteinen in Zuckerketten des Komplextyps, das die folgenden Schritte (1) und (2) umfasst:
(1) Einwirkenlassen einer Glycosidase auf Zuckerketten des Hybridtyps an Glycoproteinen; und
(2) Einwirkenlassen von β-1,2-N-Acetylglucosaminyltransferase II, bei der es sich um ein rekombinantes Fusionsprotein gemäß einem der Ansprüche 1 bis 6 handelt, auf die in Schritt (1) erhaltenen Glycoproteine in Gegenwart von UDP-GIcNAc.

50. Verfahren gemäß Anspruch 49, **dadurch gekennzeichnet, dass** nach Schritt (2) weiterhin noch wenigstens eine Art von Glycosyltransferase einwirken gelassen wird.

51. Verfahren gemäß Anspruch 50, wobei die Glycosyltransferase wenigstens eine ist, die aus Sialyltransferase, Fucosyltransferase, Galactosyltransferase und N-Acetylglucosaminyltransferase ausgewählt ist.

52. Verfahren gemäß Anspruch 50, wobei wenigstens eine Glycosyltransferase ein immobilisiertes Enzym ist.

53. Verfahren gemäß Anspruch 49, wobei die Glycosidase wenigstens eine ist, die aus Mannosidase, Xylosidase, Fucosidase und β-1,4-N-Acetylglucosaminidase ausgewählt ist.

54. Verfahren gemäß Anspruch 49, wobei wenigstens eines der Enzyme Glycosidase oder β-1,2-N-Acetylglucosaminyltransferase II ein immobilisiertes Enzym ist.

55. Verfahren gemäß Anspruch 49, wobei das Glycoprotein natürlichen Ursprungs ist.

56. Verfahren gemäß Anspruch 49, wobei das Glycoprotein rekombinant ist.

## Revendications

1. Protéine de fusion recombinante constituée d'une protéine de liaison au maltose et de la β1,2-N-acétylglucosaminyltransférase II.

2. Protéine de fusion selon la revendication 1, dans laquelle la β1,2-N-acétylglucosaminyltransférase II est d'origine humaine.

3. Protéine de fusion selon la revendication 1, dans laquelle la β1,2-N-acétylglucosaminyltransférase II est (a) une protéine comprenant la séquence d'acides aminés de SEQ ID NO:2, ou (b) une protéine ayant une activité β1,2-N-acétylglucosaminyltransférase II, comprenant la séquence d'acides aminés au point (a) ci-dessus avec un ou plusieurs acides aminés délétés, substitués ou ajoutés.

4. Protéine de fusion selon la revendication 3, dans laquelle la β1,2-N-acétylglucosaminyltransférase II comprend au moins la séquence d'acides aminés 20-447 de la séquence d'acides aminés de SEQ ID NO:2.

5. Protéine de fusion selon la revendication 1, dans laquelle la β1,2-N-acétylglucosaminyltransférase II comprend une séquence d'acides aminés dans laquelle des acides aminés correspondant à toute ou partie du domaine transmembranaire de la protéine ont été délétés.

6. Protéine de fusion selon la revendication 1, comprenant un site de reconnaissance de protéase entre la protéine de liaison au sucre et la β1,2-N-acétylglucosaminyltransférase II.

7. ADN codant pour une protéine de fusion selon l'une quelconque des revendications 1 à 6.

8. Vecteur d'expression comprenant un ADN selon la revendication 7.

9. Transformant non humain résultant d'une transformation avec un vecteur d'expression selon la revendication 8.

10. Procédé pour produire une protéine de fusion protéine de liaison au maltose/β1,2-N-acétylglucosaminyltransférase II, comprenant les étapes suivantes, consistant à:
(1) transformer *E. coli* en utilisant un vecteur d'expression auquel un ADN codant pour une protéine de liaison au maltose et un ADN codant pour la β1,2-N-acétylglucosaminyltransférase II ont été ligaturés d'une manière telle que les deux protéines sont exprimées sous forme d'une protéine de fusion sous le contrôle d'un promoteur susceptible de fonctionner dans *E. coli*;
(2) cultiver les transformants résultants pour produire une protéine de fusion constituée de la protéine de liaison au maltose et de la β1,2-N-acétylglucosaminyltransférase II; et
(3) isoler la protéine de fusion à partir de la culture résultante.

11. Procédé selon la revendication 10, dans lequel la β1,2-N-acétylglucosaminyltransférase II est d'origine humaine.

12. Procédé selon la revendication 11, dans lequel l'ADN codant pour la β1,2-N-acétylglucosaminyltransférase II comprend une séquence nucléotidique codant pour au moins la séquence d'acides aminés 29-447 de la séquence d'acides aminés de SEQ ID NO:2.

13. Procédé selon la revendication 11, dans lequel l'ADN codant pour la β1,2-N-acétylglucosaminyltransférase II comprend au moins la séquence nucléotidique 85-1341 de la séquence nucléotidique de SEQ ID NO:1.

14. Procédé selon la revendication 10, dans lequel l'ADN codant pour la β1,2-N-acétylglucosaminyltransférase II comprend une séquence nucléotidique codant pour une séquence d'acides aminés dans laquelle des acides aminés correspondant à toute ou partie du domaine transmembranaire de la protéine ont été délétés.

15. Procédé selon la revendication 10, dans lequel l'ADN codant pour la β1,2-N-acétylglucosaminyltransférase II comprend la séquence nucléotidique de SEQ ID NO:1 à partir de laquelle des acides aminés correspondant à toute ou partie du domaine transmembranaire de la protéine ont été délétés.

16. Procédé selon la revendication 10, dans lequel l'ADN codant pour la protéine de liaison au maltose est dérivé de pMAL-p2 ou pMAL-c2.

17. Procédé selon la revendication 10, dans lequel la protéine de fusion est isolée en présence d'ions métalliques divalents provenant de la culture obtenue dans l'étape (3).

18. Procédé selon la revendication 17, dans lequel le métal divalent est le manganèse.

19. Procédé pour produire de la β1,2-N-acétylglucosaminyltransférase II, comprenant l'étape consistant à isoler la β1,2-N-acétylglucosaminyltransférase II après élimination de la portion protéine de liaison au maltose de la protéine de fusion obtenue par un procédé selon l'une quelconque des revendications 10 à 18.

20. Procédé selon la revendication 19, **caractérisé en ce que** l'ADN codant pour la protéine de liaison au sucre comprend une séquence nucléotidique codant pour un site de reconnaissance de protéase à l'extrémité C-terminale de la protéine, et la portion protéine de liaison au sucre est éliminée de la protéine de fusion sous l'action d'une protéase.

21. Procédé selon la revendication 20, dans lequel la protéase est le facteur de coagulation sanguine Xa.

22. Procédé pour convertir des chaînes de sucres sur des glycoprotéines en chaînes de sucres de type complexe, comprenant les étapes (1) à (3) ci-dessous:
(1) laisser agir une glycosidase sur les chaînes de sucres des glycoprotéines;
(2) laisser agir la β1,2-N-acétylglucosaminyltransférase I, en présence d'UDP-GIcNAc, sur les glycoprotéines obtenues dans l'étape (1); et
(3) laisser agir la β1,2-N-acétylglucosaminyltransférase II, qui est une protéine de fusion recombinante selon l'une quelconque des revendications 1 à 6, en présence d'UDP-GIcNAc, sur les glycoprotéines obtenues dans l'étape (2).

23. Procédé selon la revendication 22, **caractérisé en ce qu'**on laisse en outre agir au moins un type de glycosyltransférase après l'étape (3).

24. Procédé selon la revendication 23, dans lequel la glycosyltransférase est au moins une glycosyltransférase choisie parmi la sialyltransférase, la fucosyltransférase, la galactosyltransférase et la N-acétylglucosaminyltransférase.

25. Procédé selon la revendication 23, dans lequel au moins une glycosyltransférase est une enzyme immobilisée.

26. Procédé selon la revendication 22, dans lequel la glycosidase est au moins une glycosidase choisie parmi la galactosidase, la N-acétylglucosaminidase, la fucosidase, la sialidase, la xylosidase et la mannosidase.

27. Procédé selon la revendication 22, dans lequel au moins une enzyme parmi la glycosidase, la β1,2-N-acétylglucosaminyltransférase I ou la β1,2-N-acétylglucosaminyltransférase II est une enzyme immobilisée.

28. Procédé selon la revendication 22, dans lequel la glycoprotéine est d'origine naturelle.

29. Procédé selon la revendication 22, dans lequel la glycoprotéine est recombinante.

30. Procédé selon la revendication 22, comprenant les étapes (1) à (4) ci-dessous:
(1) laisser agir une glycosidase sur les chaînes de sucres des glycoprotéines;
(2) laisser agir la β1,2-N-acétylglucosaminyltransférase I, en présence d'UDP-GIcNAc, sur les glycoprotéines obtenues dans l'étape (1);
(3) laisser agir une α-mannosidase sur les glycoprotéines obtenues dans l'étape (2); et
(4) laisser agir la β1,2-N-acétylglucosaminyltransférase II, qui est une protéine de fusion recombinante selon l'une quelconque des revendications 1 à 6, en présence d'UDP-GIcNAc, sur les glycoprotéines obtenues dans l'étape (3).

31. Procédé selon la revendication 30, **caractérisé en ce qu'**on laisse en outre agir au moins un type de glycosyltransférase après l'étape (4).

32. Procédé selon la revendication 31, dans lequel la glycosyltransférase est au moins une glycosyltransférase choisie parmi la sialyltransférase, la fucosyltransférase, la galactosyltransférase et la N-acétylglucosaminyltransférase.

33. Procédé selon la revendication 31, dans lequel au moins une glycosyltransférase est une enzyme immobilisée.

34. Procédé selon la revendication 30, dans lequel la glycosidase est au moins une glycosidase choisie parmi la galactosidase, la N-acétylglucosaminidase, la fucosidase, la sialidase, la xylosidase et la mannosidase.

35. Procédé selon la revendication 30, dans lequel la glycosidase est une α-mannosidase.

36. Procédé selon la revendication 30, dans lequel la glycosidase est une α1,2-mannosidase.

37. Procédé selon la revendication 30, dans lequel l'a-mannosidase est une α-mannosidase II.

38. Procédé selon la revendication 30, dans lequel au moins une enzyme parmi la glycosidase, la β1,2-N-acétylglucosaminyltransférase I, l'α-mannosidase ou la β1,2-N-acétylglucosaminyltransférase II est une enzyme immobilisée.

39. Procédé selon la revendication 30, dans lequel la glycoprotéine est d'origine naturelle.

40. Procédé selon la revendication 30, dans lequel la glycoprotéine est recombinante.

41. Procédé pour convertir des chaînes de sucres sur des glycoprotéines en chaînes de sucres de type complexe, comprenant les étapes (1) à (3) suivantes:
(1) laisser agir la β1,2-N-acétylglucosaminyltransférase I, en présence d'UDP-GIcNAc, sur des glycoprotéines ayant une structure dans laquelle toute ou partie des structures des chaînes de sucres sur les glycoprotéines servent de substrat à la β1,2-N-acétylglucosaminyltransférase I;
(2) laisser agir une α-mannosidase sur les glycoprotéines obtenues dans l'étape (1); et
(3) laisser agir la β1,2-N-acétylglucosaminyltransférase II, qui est une protéine de fusion recombinante selon l'une quelconque des revendications 1 à 6, en présence d'UDP-GIcNAc, sur les glycoprotéines obtenues dans l'étape (2).

42. Procédé selon la revendication 41 pour convertir des chaînes de sucres sur des glycoprotéines en chaînes de sucres de type complexe, **caractérisé en ce qu'**on laisse en outre agir au moins un type de glycosyltransférase après l'étape (3).

43. Procédé selon la revendication 42, dans lequel la glycosyltransférase est au moins une glycosyltransférase choisie parmi la sialyltransférase, la fucosyltransférase, la galactosyltransférase et la N-acétylglucosaminyltransférase.

44. Procédé selon la revendication 42, dans lequel au moins une glycosyltransférase est une enzyme immobilisée.

45. Procédé selon la revendication 41, dans lequel l'a-mannosidase est une α-mannosidase II.

46. Procédé selon la revendication 41, dans lequel au moins une enzyme parmi la β1,2-N-acétylglucosaminyltransférase I, l'α-mannosidase ou la β1,2-N-acétylglucosaminyltransférase II est une enzyme immobilisée.

47. Procédé selon la revendication 41, dans lequel la glycoprotéine est d'origine naturelle.

48. Procédé selon la revendication 41, dans lequel la glycoprotéine est recombinante.

49. Procédé pour convertir des chaînes de sucres de type hybride sur des glycoprotéines en chaînes de sucres de type complexe, comprenant les étapes (1) et (2) ci-dessous:
(1) laisser agir une glycosidase sur des chaînes de sucres de type hybride de glycoprotéines, et
(2) laisser agir la β1,2-N-acétylglucosaminyltransférase II, qui est une protéine de fusion recombinante selon l'une quelconque des revendications 1 à 6, en présence d'UDP-GIcNAc, sur les glycoprotéines obtenues dans l'étape (1).

50. Procédé selon la revendication 49, **caractérisé en ce qu'**on laisse en outre agir au moins un type de glycosyltransférase après l'étape (2).

51. Procédé selon la revendication 50, dans lequel la glycosyltransférase est au moins une glycosyltransférase choisie parmi la sialyltransférase, la fucosyltransférase, la galactosyltransférase et la N-acétylglucosaminyltransférase.

52. Procédé selon la revendication 50, dans lequel au moins une glycosyltransférase est une enzyme immobilisée.

53. Procédé selon la revendication 49, dans lequel la glycosidase est au moins une glycosidase choisie parmi la mannosidase, la xylosidase, la fucosidase et la β1,4-N-acétylglucosaminidase.

54. Procédé selon la revendication 49, dans lequel au moins une enzyme parmi la glycosidase ou la β1,2-N-acétylglucosaminyltransférase II est une enzyme immobilisée.

55. Procédé selon la revendication 49, dans lequel la glycoprotéine est d'origine naturelle.

56. Procédé selon la revendication 49, dans lequel la glycoprotéine est recombinante.


### SEQUENCE LISTING

<110> Toyo Boseki Kabushiki Kaisha
   Fujiyama, Kazuhito
   Seki, Tatsuji
<120> A novel fusion protein of beta 1,2-N-acetylglucosaminyl transferase II and preparation thereof
<130> P02-08
<140>
   <141>
<150> JP2001-49955
   <151> 2001-02-26
<150> JP2001-250165
   <151> 2001-08-21
<160> 9
<170> PatentIn Ver. 2.1
<210> 1
   <211> 1392
   <212> DNA
   <213> Homosapiens
<220>
   <221> CDS
   <222> (1).. (1344)
<400> 1
   atg agg ttc cgc atc tac aaa cgg aag gtg cta atc ctg acg ctc gtg 48
   Met Arg Phe Arg Ile Tyr Lys Arg Lys Val Leu Ile Leu Thr Leu Val
   1 5 10 15
   gtg gcc gcc tgc ggc ttc gtc ctc tgg agc agc aat ggg cga caa agg 96
   Val Ala Ala Cys Gly Phe Val Leu Trp Ser Ser Asn Gly Arg Gln Arg
   20 25 30
   aag aac gag gcc ctc gcc cca ccg ttg ctg gac gcc gaa ccc gcg cgg 144
   Lys Asn Glu Ala Leu Ala Pro Pro Leu Leu Asp Ala Glu Pro Ala Arg
   35 40 45
   ggt gcc ggc ggc cgc ggt ggg gac cac ccc tct gtg gct gtg ggc atc 192
   Gly Ala Gly Gly Arg Gly Gly Asp His Pro Ser Val Ala Val Gly Ile
   50 55 60
   cgc agg gtc tcc aac gtg tcg gcg gct tcc ctg gtc ccg gcg gtc ccc 240
   Arg Arg Val Ser Asn Val Ser Ala Ala Ser Leu Val Pro Ala Val Pro
   65 70 75 80
   cag ccc gag gcg gac aac ctg acg ctg cgg tac cgg tcc ctg gtg tac 288
   Gln Pro Glu Ala Asp Asn Leu Thr Leu Arg Tyr Arg Ser Leu Val Tyr
   85 90 95
   cag ctg aac ttt gat cag acc ctg agg aat gta gat aag gct ggc acc 336
   Gln Leu Asn Phe Asp Gln Thr Leu Arg Asn Val Asp Lys Ala Gly Thr
   100 105 110
   tgg gcc ccc cgg gag ctg gtg ctg gtg gtc cag gtg cat aac cgg ccc 384
   Trp Ala Pro Arg Glu Leu Val Leu Val Val Gln Val His Asn Arg Pro
   115 120 125
   gaa tac ctc aga ctg ctg ctg gac tca ctt cga aaa gcc cag gga att 432
   Glu Tyr Leu Arg Leu Leu Leu Asp Ser Leu Arg Lys Ala Gln Gly Ile
   130 135 140
   gac aac gtc ctc gtc atc ttt agc cat gac ttc tgg tcg acc gag atc 480
   Asp Asn Val Leu Val Ile Phe Ser His Asp Phe Trp Ser Thr Glu Ile
   145 150 155 160
   aat cag ctg atc gcc ggg gtg aat ttc tgt ccg gtt ctg cag gtg ttc 528
   Asn Gln Leu Ile Ala Gly Val Asn Phe Cys Pro Val Leu Gln Val Phe
   165 170 175
   ttt cct ttc agc att cag ttg tac cct aac gag ttt cca ggt agt gac 576
   Phe Pro Phe Ser Ile Gln Leu Tyr Pro Asn Glu Phe Pro Gly Ser Asp
   180 185 190
   cct aga gat tgt ccc aga gac ctg ccg aag aat gcc gct ttg aaa ttg 624
   Pro Arg Asp Cys Pro Arg Asp Leu Pro Lys Asn Ala Ala Leu Lys Leu
   195 200 205
   ggg tgc atc aat gct gag tat ccc gac tcc ttc ggc cat tat aga gag 672
   Gly Cys Ile Asn Ala Glu Tyr Pro Asp Ser Phe Gly His Tyr Arg Glu
   210 215 220
   gcc aaa ttc tcc cag acc aaa cat cac tgg tgg tgg aag ctg cat ttt 720
   Ala Lys Phe Ser Gln Thr Lys His His Trp Trp Trp Lys Leu His Phe
   225 230 235 240
   gtg tgg gaa aga gtg aaa att ctt cga gat tat gct ggc ctt ata ctt 768
   Val Trp Glu Arg Val Lys Ile Leu Arg Asp Tyr Ala Gly Leu Ile Leu
   245 250 255
   ttc cta gaa gag gat cac tac tta gcc cca gac ttt tac cat gtc ttc 816
   Phe Leu Glu Glu Asp His Tyr Leu Ala Pro Asp Phe Tyr His Val Phe
   260 265 270
   aaa aag atg tgg aaa ctg aag cag caa gag tgc cct gaa tgt gat gtt 864
   Lys Lys Met Trp Lys Leu Lys Gln Gln Glu Cys Pro Glu Cys Asp Val
   275 280 285
   ctc tcc ctg ggg acc tat agt gcc agt cgc agt ttc tat ggc atg gct 912
   Leu Ser Leu Gly Thr Tyr Ser Ala Ser Arg Ser Phe Tyr Gly Met Ala
   290 295 300
   gac aag gta gat gtg aaa act tgg aaa tcc aca gag cac aat atg ggt 960
   Asp Lys Val Asp Val Lys Thr Trp Lys Ser Thr Glu His Asn Met Gly
   305 310 315 320
   cta gcc ttg acc cgg aat gcc tat cag aag ctg atc gag tgc aca gac 1008
   Leu Ala Leu Thr Arg Asn Ala Tyr Gln Lys Leu Ile Glu Cys Thr Asp
   325 330 335
   act ttc tgt act tat gat gat tat aac tgg gac tgg act ctt caa tac 1056
   Thr Phe Cys Thr Tyr Asp Asp Tyr Asn Trp Asp Trp Thr Leu Gln Tyr
   340 345 350
   ttg act gta tct tgt ctt cca aaa ttc tgg aaa gtg ctg gtt cct caa 1104
   Leu Thr Val Ser Cys Leu Pro Lys Phe Trp Lys Val Leu Val Pro Gln
   355 360 365
   att cct agg atc ttt cat gct gga gac tgt ggt atg cat cac aag aaa 1152
   Ile Pro Arg Ile Phe His Ala Gly Asp Cys Gly Met His His Lys Lys
   370 375 380
   acc tgt aga cca tcc act cag agt gcc caa att gag tca ctc tta aat 1200
   Thr Cys Arg Pro Ser Thr Gln Ser Ala Gln Ile Glu Ser Leu Leu Asn
   385 390 395 400
   aat aac aaa caa tac atg ttt cca gaa act cta act atc agt gaa aag 1248
   Asn Asn Lys Gln Tyr Met Phe Pro Glu Thr Leu Thr Ile Ser Glu Lys
   405 410 415
   ttt act gtg gta gcc att tcc cca cct aga aaa aat gga ggg tgg gga 1296
   Phe Thr Val Val Ala Ile Ser Pro Pro Arg Lys Asn Gly Gly Trp Gly
   420 425 430
   gat att agg gac cat gaa ctc tgt aaa agt tat aga aga ctg cag tga 1344
   Asp Ile Arg Asp His Glu Leu Cys Lys Ser Tyr Arg Arg Leu Gln
   435 440 445
   aaatcacagt tacaaaagcg acagtcttct atttttgata tttgtcca 1392
<210> 2
   <211> 447
   <212> PRT
   <213> Homosapiens
<400> 2
   Met Arg Phe Arg Ile Tyr Lys Arg Lys Val Leu Ile Leu Thr Leu Val
   1 5 10 15
   Val Ala Ala Cys Gly Phe Val Leu Trp Ser Ser Asn Gly Arg Gln Arg
   20 25 30
   Lys Asn Glu Ala Leu Ala Pro Pro Leu Leu Asp Ala Glu Pro Ala Arg
   35 40 45
   Gly Ala Gly Gly Arg Gly Gly Asp His Pro Ser Val Ala Val Gly Ile
   50 55 60
   Arg Arg Val Ser Asn Val Ser Ala Ala Ser Leu Val Pro Ala Val Pro
   65 70 75 80
   Gln Pro Glu Ala Asp Asn Leu Thr Leu Arg Tyr Arg Ser Leu Val Tyr
   85 90 95
   Gln Leu Asn Phe Asp Gln Thr Leu Arg Asn Val Asp Lys Ala Gly Thr
   100 105 110
   Trp Ala Pro Arg Glu Leu Val Leu Val Val Gln Val His Asn Arg Pro
   115 120 125
   Glu Tyr Leu Arg Leu Leu Leu Asp Ser Leu Arg Lys Ala Gln Gly Ile
   130 135 140
   Asp Asn Val Leu Val Ile Phe Ser His Asp Phe Trp Ser Thr Glu Ile
   145 150 155 160
   Asn Gln Leu Ile Ala Gly Val Asn Phe Cys Pro Val Leu Gln Val Phe
   165 170 175
   Phe Pro Phe Ser Ile Gln Leu Tyr Pro Asn Glu Phe Pro Gly Ser Asp
   180 185 190
   Pro Arg Asp Cys Pro Arg Asp Leu Pro Lys Asn Ala Ala Leu Lys Leu
   195 200 205
   Gly Cys Ile Asn Ala Glu Tyr Pro Asp Ser Phe Gly His Tyr Arg Glu
   210 215 220
   Ala Lys Phe Ser Gln Thr Lys His His Trp Trp Trp Lys Leu His Phe
   225 230 235 240
   Val Trp Glu Arg Val Lys Ile Leu Arg Asp Tyr Ala Gly Leu Ile Leu
   245 250 255
   Phe Leu Glu Glu Asp His Tyr Leu Ala Pro Asp Phe Tyr His Val Phe
   260 265 270
   Lys Lys Met Trp Lys Leu Lys Gln Gln Glu Cys Pro Glu Cys Asp Val
   275 280 285
   Leu Ser Leu Gly Thr Tyr Ser Ala Ser Arg Ser Phe Tyr Gly Met Ala
   290 295 300
   Asp Lys Val Asp Val Lys Thr Trp Lys Ser Thr Glu His Asn Met Gly
   305 310 315 320
   Leu Ala Leu Thr Arg Asn Ala Tyr Gln Lys Leu Ile Glu Cys Thr Asp
   325 330 335
   Thr Phe Cys Thr Tyr Asp Asp Tyr Asn Trp Asp Trp Thr Leu Gln Tyr
   340 345 350
   Leu Thr Val Ser Cys Leu Pro Lys Phe Trp Lys Val Leu Val Pro Gln
   355 360 365
   Ile Pro Arg Ile Phe His Ala Gly Asp Cys Gly Met His His Lys Lys
   370 375 380
   Thr Cys Arg Pro Ser Thr Gln Ser Ala Gln Ile Glu Ser Leu Leu Asn
   385 390 395 400
   Asn Asn Lys Gln Tyr Met Phe Pro Glu Thr Leu Thr Ile Ser Glu Lys
   405 410 415
   Phe Thr Val Val Ala Ile Ser Pro Pro Arg Lys Asn Gly Gly Trp Gly
   420 425 430
   Asp Ile Arg Asp His Glu Leu Cys Lys Ser Tyr Arg Arg Leu Gln
   435 440 445
<210> 3
   <211> 4
   <212> PRT
   <213> Homosapiens
<400> 3
   Ile Glu Gly Arg
   1
<210> 4
   <211> 24
   <212> DNA
   <213> Artificial Sequence
<220>
   <223> Description of Artificial Sequence:Primer
<400> 4
   aggttccgca tctacaaacg gaag 24
<210> 5
   <211> 27
   <212> DNA
   <213> Artificial Sequence
<220>
   <223> Description of Artificial Sequence:Primer
<400> 5
   tgtcgctttt gtaactgtga ttttcac 27
<210> 6
   <211> 29
   <212> DNA
   <213> Artificial Sequence
<220>
   <223> Description of Artificial Sequence:Primer
<400> 6
   aaggatccgg gcgacaaagg aagaacgag 29
<210> 7
   <211> 32
   <212> DNA
   <213> Artificial Sequence
<220>
   <223> Description of Artificial Sequence:Primer
<400> 7
   aaaagcttgt aactgtgatt ttcactgcag tc 32
<210> 8
   <211> 39
   <212> DNA
   <213> Artificial Sequence
<220>
   <223> Description of Artificial Sequence:Spacer
<220>
   <221> CDS
   <222> (1).. (39)
<400> 8
   tcg agc tcg aac aac aac aac aat aac aat aac aac aac          39
   Ser Ser Ser Asn Asn Asn Asn Asn Asn Asn Asn Asn Asn
    1               5                  10
<210> 9
   <211> 13
   <212> PRT
   <213> Artificial Sequence
   <223> Description of Artificial Sequence:Spacer
<400> 9
   Ser Ser Ser Asn Asn Asn Asn Asn Asn Asn Asn Asn Asn
    1               5                  10